# EUROPEAN PATENT SPECIFICATION

(11) **EP 4 129 199 B1**
(45) Date of publication and mention of the grant of the patent: **02.09.2026**
(21) Application number: 21775972.9
(22) Date of filing: 01.03.2021
(51) Int. Cl.: A61B 8/14, A61B 8/08

(54) **ULTRASONIC DIAGNOSTIC APPARATUS, CONTROL METHOD FOR ULTRASONIC DIAGNOSTIC APPARATUS, AND PROCESSOR FOR ULTRASONIC DIAGNOSTIC APPARATUS**
ULTRASCHALLDIAGNOSEVORRICHTUNG, STEUERUNGSVERFAHREN FÜR DIE ULTRASCHALLDIAGNOSEVORRICHTUNG UND PROZESSOR FÜR DIE ULTRASCHALLDIAGNOSEVORRICHTUNG
APPAREIL DE DIAGNOSTIC À ULTRASONS, PROCÉDÉ DE COMMANDE D'APPAREIL DE DIAGNOSTIC À ULTRASONS, ET PROCESSEUR D'APPAREIL DE DIAGNOSTIC À ULTRASONS

(30) Priority: 24.03.2020 JP 2020052268
(43) Date of publication of application: 08.02.2023
(73) Proprietor: FUJIFILM Corporation, Tokyo 106-8620 (JP)
(72) Inventor: EBATA Tetsurou, Ashigarakami-gun, Kanagawa 258-8538 (JP)
(74) Representative: Dehns Germany Partnerschaft mbB
(86) International application number: PCT/JP2021/007663
(87) International publication number: WO 2021/192824

(56) References cited:
- EP-A1- 3 549 528
- WO-A1-2020/016018
- JP-A- 2007 117 433
- JP-A- 2007 117 433
- JP-A- 2009 183 571
- JP-A- 2009 279 435
- US-A1- 2016 192 904
- US-A1- 2017 219 705

## Description

### BACKGROUND OF THE INVENTION

### 1. Field of the Invention

The present invention relates to an ultrasound diagnostic apparatus for measuring a urine volume in a urinary bladder of a subject, a method for controlling the ultrasound diagnostic apparatus, and a processor for the ultrasound diagnostic apparatus.

### 2. Description of the Related Art

In the related art, an ultrasound diagnostic apparatus is used to observe the urinary bladder of a subject and measure the urine volume in the observed urinary bladder. In general, the urine volume in the urinary bladder of a subject is substantially equal to the volume of the urinary bladder of the subject. Thus, the volume of the urinary bladder of the subject is measured as the urine volume. To perform such urine volume measurement easily, for example, an ultrasound diagnostic apparatus of JP2017-109074A has been developed. In response to a user pressing a trigger button included in the ultrasound diagnostic apparatus of JP2017-109074A in a state in which ultrasound images of a plurality of frames including the urinary bladder of a subject are acquired, the ultrasound diagnostic apparatus automatically selects an ultrasound image of a frame determined to be suitable for urine volume measurement, based on the ultrasound images of the plurality of frames acquired by the user. The ultrasound diagnostic apparatus then measures the urine volume based on the ultrasound image of the selected frame.

Further relevant prior art systems and methods are disclosed in the patent documents EP 3 549 528 A1, WO 2020/016018 A1, JP 2007 117433 A and US 2017/219705 A1.

### SUMMARY OF THE INVENTION

However, the ultrasound diagnostic apparatus of JP2017-109074A may erroneously detect a urinary bladder region included in the ultrasound images and thus may select an ultrasound image of a frame not suitable for urine volume measurement such as a frame in which the diameter of the urinary bladder is erroneously measured. Consequently, the accuracy of urine volume measurement may decrease.

When the user attempts to manually select an ultrasound image of a frame subjected to urine volume measurement in order to select the ultrasound image of the suitable frame, the user needs to check the ultrasound images of many frames. This requires a great deal of labor.

Accordingly, for example, it is conceivable that the ultrasound diagnostic apparatus presents, to the user, a plurality of candidate frames that serve as candidates for the ultrasound image of the frame to be used for urine volume measurement from the ultrasound images of the plurality of frames, and the user selects the ultrasound image of the frame to be used for urine volume measurement from the plurality of candidate frames. However, the ultrasound images of the plurality of frames acquired consecutively in time series may include many similar ultrasound images, and the ultrasound images of the frames similar to each other may be presented to the user as the ultrasound images of the candidate frames.

In this case, it is expectedly difficult particularly for a user not having sufficient skill and expertise to select the ultrasound image of the frame suitable for urine volume measurement from the ultrasound images of the plurality of candidate frames similar to each other.

The present invention is made to overcome such issues in the related art, and an object thereof is to provide an ultrasound diagnostic apparatus capable of increasing the accuracy of urine volume measurement while reducing a load imposed on a user, a method for controlling the ultrasound diagnostic apparatus, and a processor for the ultrasound diagnostic apparatus.

To achieve this object, an ultrasound diagnostic apparatus is provided as defined by claim 1.

The frame thinning-out unit can extract an ultrasound image of a frame at predetermined intervals in time series from the ultrasound images of the first frame group and remove ultrasound images of rest of the frames to form the second frame group of ultrasound images.

The predetermined intervals are intervals of a predetermined number of frames or predetermined time intervals.

The similarity estimation unit can include a similarity level calculation unit configured to analyze the ultrasound images of the two frames to calculate a similarity level between the ultrasound images of the two frames, and can estimate that the ultrasound images of the two frames are similar to each other in a case where the similarity level calculated by the similarity level calculation unit is greater than or equal to a predetermined similarity level threshold, and estimate that the ultrasound images of the two frames are dissimilar from each other in a case where the similarity level calculated by the similarity level calculation unit is less than the predetermined similarity level threshold.

Alternatively, the similarity estimation unit may include an area ratio calculation unit configured to analyze the ultrasound images of the two frames to calculate an area ratio between urinary bladder regions in the respective ultrasound images of the two frames, and may estimate that the ultrasound images of the two frames are similar to each other in a case where the area ratio calculated by the area ratio calculation unit is within a predetermined area ratio range, and estimate that the ultrasound images of the two frames are dissimilar from each other in a case where the area ratio calculated by the area ratio calculation unit is out of the predetermined area ratio range.

Alternatively, the ultrasound diagnostic apparatus may further include an ultrasound probe configured to transmit an ultrasonic beam to a subject and receive an ultrasonic beam from the subject, and an angle sensor attached to the ultrasound probe and configured to detect an angle of the ultrasound probe. The similarity estimation unit may include a change-in-angle calculation unit configured to calculate, based on the angle of the ultrasound probe detected by the angle sensor, a change in the angle of the ultrasound probe during capturing of the ultrasound images of the two frames, and may estimate that the ultrasound images of the two frames are similar to each other in a case where the change in the angle calculated by the change-in-angle calculation unit is smaller than a predetermined change-in-angle threshold, and estimate that the ultrasound images of the two frames are dissimilar from each other in a case where the change in the angle calculated by the change-in-angle calculation unit is greater than or equal to the predetermined change-in-angle threshold.

The frame thinning-out unit can update the predetermined intervals, based on the similarity level calculated by the similarity level calculation unit, the area ratio calculated by the area ratio calculation unit, or the change in the angle calculated by the change-in-angle calculation unit.

The frame thinning-out unit can update the predetermined intervals, based on a processing speed of the ultrasound diagnostic apparatus.

The frame thinning-out unit can update the predetermined intervals, based on a time taken for scanning of a urinary bladder.

The frame thinning-out unit can include a similarity estimation unit configured to estimate whether or not ultrasound images of two frames are similar to each other, and can form the second frame group of ultrasound images using ultrasound images of frames that are left after removal of an ultrasound image of a frame that is consecutive to an ultrasound image of each frame of the ultrasound images of the first frame group and is estimated to be similar to the ultrasound image of the frame by the similarity estimation unit.

In this case, the similarity estimation unit can include a similarity level calculation unit configured to analyze the ultrasound images of the two frames to calculate a similarity level between the ultrasound images of the two frames, and can estimate that the ultrasound images of the two frames are similar to each other in a case where the similarity level calculated by the similarity level calculation unit is greater than or equal to a predetermined similarity level threshold, and estimate that the ultrasound images of the two frames are dissimilar from each other in a case where the similarity level calculated by the similarity level calculation unit is less than the predetermined similarity level threshold.

Alternatively, the similarity estimation unit may include an area ratio calculation unit configured to analyze the ultrasound images of the two frames to calculate an area ratio between urinary bladder regions in the respective ultrasound images of the two frames, and may estimate that the ultrasound images of the two frames are similar to each other in a case where the area ratio calculated by the area ratio calculation unit is within a predetermined area ratio range, and estimate that the ultrasound images of the two frames are dissimilar from each other in a case where the area ratio calculated by the area ratio calculation unit is out of the predetermined area ratio range.

Alternatively, the ultrasound diagnostic apparatus may further include an ultrasound probe configured to transmit an ultrasonic beam to a subject and receive an ultrasonic beam from the subject, and an angle sensor attached to the ultrasound probe and configured to detect an angle of the ultrasound probe. The similarity estimation unit may include a change-in-angle calculation unit configured to calculate, based on the angle of the ultrasound probe detected by the angle sensor, a change in the angle of the ultrasound probe during capturing of the ultrasound images of the two frames, and may estimate that the ultrasound images of the two frames are similar to each other in a case where the change in the angle calculated by the change-in-angle calculation unit is smaller than a predetermined change-in-angle threshold, and estimate that the ultrasound images of the two frames are dissimilar from each other in a case where the change in the angle calculated by the change-in-angle calculation unit is greater than or equal to the predetermined angle-of-change threshold.

A method for controlling an ultrasound diagnostic apparatus is provided by claim 12.

A processor for controlling an ultrasound diagnostic apparatus is provided by claim 13.

According to the present invention, the ultrasound diagnostic apparatus includes the frame thinning-out unit configured to thin out ultrasound images of some frames from ultrasound images of a first frame group to form a second frame group of ultrasound images, the second frame group being constituted by a smaller number of frames than the first frame group; the feature quantity calculation unit configured to calculate a feature quantity related to a urinary bladder region extracted for each of the ultrasound images of the second frame group; the candidate frame extraction unit configured to extract, based on the feature quantity calculated by the feature quantity calculation unit, an ultrasound image of at least one candidate frame that serves as a candidate subjected to measurement, from the ultrasound images of the second frame group; the measurement frame selection unit configured to select, in accordance with an input operation performed by a user via an input device, an ultrasound image of a measurement frame that serves as a target subjected to measurement, from the ultrasound image of the at least one candidate frame displayed on a monitor; and the urine volume measurement unit configured to analyze the ultrasound image of the measurement frame selected by the measurement frame selection unit to measure a urine volume. Thus, the ultrasound diagnostic apparatus can increase the accuracy in urine volume measurement while reducing the load imposed on the user.

### BRIEF DESCRIPTION OF THE DRAWINGS

Fig. 1 is a block diagram illustrating a configuration of an ultrasound diagnostic apparatus according to a first embodiment of the present invention;
Fig. 2 is a block diagram illustrating an internal configuration of a transmission/reception circuit in the first embodiment of the present invention;
Fig. 3 is a block diagram illustrating an internal configuration of an image generation unit in the first embodiment of the present invention;
Fig. 4 is a diagram schematically illustrating an example of an ultrasound image including a urinary bladder region in the first embodiment of the present invention;
Fig. 5 is a diagram schematically illustrating an example of ultrasound images of a plurality of candidate frames displayed in the first embodiment of the present invention;
Fig. 6 is a diagram illustrating an example of an ellipsoid;
Fig. 7 is a flowchart illustrating an operation of the ultrasound diagnostic apparatus according to the first embodiment of the present invention;
Fig. 8 is a diagram schematically illustrating an example in which a urine volume in a urinary bladder of a subject is displayed on a monitor in the first embodiment of the present invention;
Fig. 9 is a block diagram illustrating an internal configuration of a frame thinning-out unit in a second embodiment of the present invention;
Fig. 10 is a flowchart illustrating an operation of forming a second frame group of ultrasound images from a first frame group of ultrasound images in the second embodiment of the present invention;
Fig. 11 is a block diagram illustrating an internal configuration of a frame thinning-out unit in a third embodiment of the present invention;
Fig. 12 is a block diagram illustrating a configuration of an ultrasound diagnostic apparatus according to a fourth embodiment of the present invention;
Fig. 13 is a diagram schematically illustrating an ultrasound probe that is in contact with a body surface of a subject;
Fig. 14 is a block diagram illustrating an internal configuration of a frame thinning-out unit in the fourth embodiment of the present invention;
Fig. 15 is a block diagram illustrating an internal configuration of a frame thinning-out unit in a fifth embodiment of the present invention;
Fig. 16 is a block diagram illustrating an internal configuration of a frame thinning-out unit in a sixth embodiment of the present invention;
Fig. 17 is a block diagram illustrating an internal configuration of a frame thinning-out unit according to a seventh embodiment of the present invention;
Fig. 18 is a block diagram illustrating an internal configuration of a frame thinning-out unit in an eighth embodiment of the present invention;
Fig. 19 is a block diagram illustrating an internal configuration of a frame thinning-out unit in a ninth embodiment of the present invention;
Fig. 20 is a diagram schematically illustrating how a urinary bladder is scanned using a swing method;
Fig. 21 is a diagram illustrating an example of a time-series change of an area of a urinary bladder region;
Fig. 22 is a diagram schematically illustrating how a urinary bladder is scanned using a slide method; and
Fig. 23 is a block diagram illustrating a configuration of an ultrasound diagnostic apparatus according to a tenth embodiment of the present invention.

### DESCRIPTION OF THE PREFERRED EMBODIMENTS

Embodiments of this invention will be described below with reference to the accompanying drawings.

The description of constituent elements below is given based on representative embodiments of the present invention. However, the present invention is not limited to such embodiments.

In the present specification, a numerical range expressed using "to" means a range including a numerical value preceding "to" as a lower limit value and a numerical value following "to" as an upper limit value.

In the present specification, the terms "identical" and "same" include an error range generally acceptable in the technical field.

### First Embodiment

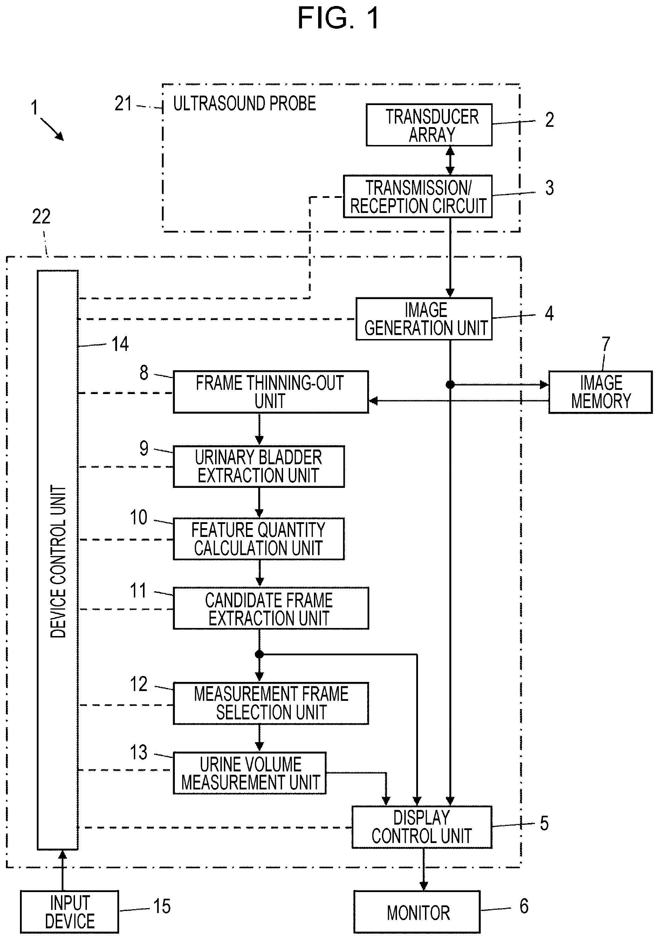

Fig. 1 illustrates a configuration of an ultrasound diagnostic apparatus 1 according to a first embodiment of the present invention. The ultrasound diagnostic apparatus 1 includes a transducer array 2. A transmission/reception circuit 3, an image generation unit 4, a display control unit 5, and a monitor 6 are sequentially connected to the transducer array 2. The transducer array 2 and the transmission/reception circuit 3 are included in an ultrasound probe 21. An image memory 7 is connected to the image generation unit 4. A frame thinning-out unit 8 is connected to the image memory 7. A urinary bladder extraction unit 9, a feature quantity calculation unit 10, a candidate frame extraction unit 11, a measurement frame selection unit 12, and a urine volume measurement unit 13 are sequentially connected to the frame thinning-out unit 8. The candidate frame extraction unit 11 and the urine volume measurement unit 13 are connected to the display control unit 5.

A device control unit 14 is connected to the transmission/reception circuit 3, the image generation unit 4, the display control unit 5, the frame thinning-out unit 8, the urinary bladder extraction unit 9, the feature quantity calculation unit 10, the candidate frame extraction unit 11, the measurement frame selection unit 12, and the urine volume measurement unit 13. An input device 15 is connected to the device control unit 14.

The image generation unit 4, the display control unit 5, the frame thinning-out unit 8, the urinary bladder extraction unit 9, the feature quantity calculation unit 10, the candidate frame extraction unit 11, the measurement frame selection unit 12, the urine volume measurement unit 13, and the device control unit 14 constitute a processor 22 for the ultrasound diagnostic apparatus 1.

The transducer array 2 of the ultrasound probe 21 illustrated in Fig. 1 has a plurality of transducers arranged one-dimensionally or two-dimensionally. Each of these transducers transmits an ultrasonic wave in accordance with a drive signal supplied from the transmission/reception circuit 3. Each of these transducers also receives an ultrasonic echo from the subject, and outputs a signal based on the ultrasonic echo. Each of the transducers is configured by forming electrodes at respective ends of a piezoelectric body constituted by, for example, a piezoelectric ceramic represented by PZT (lead zirconate titanate), a polymer piezoelectric element represented by PVDF (polyvinylidene difluoride), and a piezoelectric single crystal represented by PMN-PT (lead magnesium niobate-lead titanate).

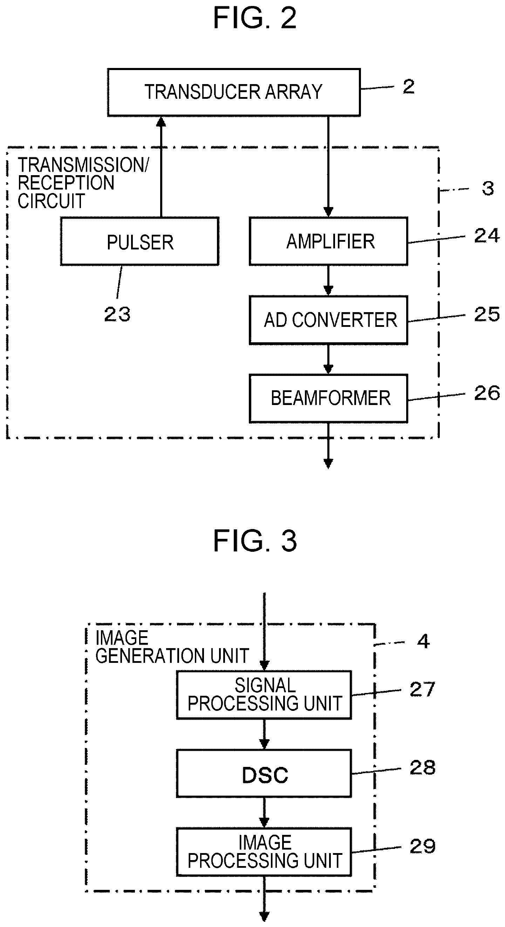

Under the control of the device control unit 14, the transmission/reception circuit 3 transmits ultrasonic waves from the transducer array 2, and generates a sound ray signal based on reception signals acquired by the transducer array 2. As illustrated in Fig. 2, the transmission/reception circuit 3 has a pulser 23 connected to the transducer array 2, and an amplifier 24, an analog-to-digital (AD) converter 25, and a beamformer 26 sequentially connected in series from the transducer array 2.

The pulser 23 includes, for example, a plurality of pulse generators. The pulser 23 adjusts delay amounts of respective drive signals, based on a transmission delay pattern selected in accordance with a control signal from the device control unit 14 so that ultrasonic waves transmitted from the plurality of transducers of the transducer array 2 form an ultrasonic beam, and supplies the resulting drive signals to the respective transducers. As described above, in response to application of a pulsed or continuous-wave voltage to the electrodes of the transducers of the transducer array 2, the piezoelectric body expands and contracts. Consequently, pulsed or continuous-wave ultrasonic waves are generated from the respective transducers, and an ultrasonic beam is formed from a composite wave of those ultrasonic waves.

The transmitted ultrasonic beam is reflected by a target such as a part of a subject, for example, and propagates toward the transducer array 2 of the ultrasound probe 21. An ultrasonic echo propagating toward the transducer array 2 in this manner is received by each of the transducers of the transducer array 2. At this time, in response to receipt of the propagating ultrasonic echo, the transducers of the transducer array 2 expand and contract to generate respective reception signals, which are electric signals, and output these reception signals to the amplifier 24.

The amplifier 24 amplifies a signal input from each of the transducers of the transducer array 2, and transmits the amplified signal to the AD converter 25. The AD converter 25 converts the signals transmitted from the amplifier 24 into pieces of digital reception data, and transmits these pieces of reception data to the beamformer 26. The beamformer 26 performs so-called reception focusing processing in which the pieces of reception data obtained by the AD converter 25 through conversion are given respective delays and then are added in accordance with sonic velocities or a sonic velocity distribution set based on the reception delay pattern selected in accordance with the control signal from the device control unit 14. Through this reception focusing processing, the pieces of reception data obtained by the AD converter 25 through the conversion are subjected to phasing addition, and a sound ray signal to which the focus of the ultrasonic echo converges is acquired.

As illustrated in Fig. 3, the image generation unit 4 has a configuration in which a signal processing unit 27, a digital scan converter (DSC) 28, and an image processing unit 29 are sequentially connected in series.

In accordance with a depth of a reflected position of the ultrasonic waves, the signal processing unit 27 performs distance-based attenuation correction on the sound ray signal generated by the beamformer 26 of the transmission/reception circuit 3, and then performs envelope detection processing. In this manner, the signal processing unit 27 generates a B-mode image signal, which is tomographic image information related to a tissue in the subject.

The DSC 28 converts (raster-converts) the B-mode image signal generated by the signal processing unit 27 into an image signal according to a normal television signal scanning method.

The image processing unit 29 performs various kinds of necessary image processing such as grayscale processing on the B-mode image signal input from the DSC 28, and then outputs the B-mode image signal to the display control unit 5 and the image memory 7. Hereinafter, the B-mode image signal on which the image processing has been performed by the image processing unit 29 is simply referred to as an ultrasound image.

The image memory 7 is a memory that stores ultrasound images of a plurality of frames. For example, the image memory 7 can store ultrasound images of a series of frames generated for each diagnosis by the image generation unit 4, as a first frame group of ultrasound images.

The image memory 7 to be used may be a recording medium such as a flash memory, a hard disc drive (HDD), a solid state drive (SDD), a flexible disc (FD), a magneto-optical disc (MO), a magnetic tape (MT), a random access memory (RAM), a compact disc (CD), a digital versatile disc (DVD), a secure digital (SD) card, a Universal Serial Bus (USB) memory; a server; or the like.

The frame thinning-out unit 8 extracts, based on a set condition, an ultrasound image of at least one frame from the ultrasound images of the first frame group stored in the image memory 7 and deletes the ultrasound images of the rest of the frames not extracted, to thin out ultrasound images of some frames from the ultrasound images of the first frame group and form a second frame group of ultrasound images. The second frame group is constituted by ultrasound images of a smaller number of frames than the ultrasound images of the first frame group. For example, the frame thinning-out unit 8 extracts an ultrasound image at intervals of a predetermined number of frames in time series from the ultrasound images of the first frame group. In this manner, the frame thinning-out unit 8 can extract the ultrasound images of the plurality of frames not adjacent to each other in time series and use these ultrasound images as ultrasound images of the second frame group. Alternatively, for example, the frame thinning-out unit 8 may extract an ultrasound image at predetermined time intervals in time series from the ultrasound images of the first frame group, and may use the extracted ultrasound images as the ultrasound images of the second frame group.

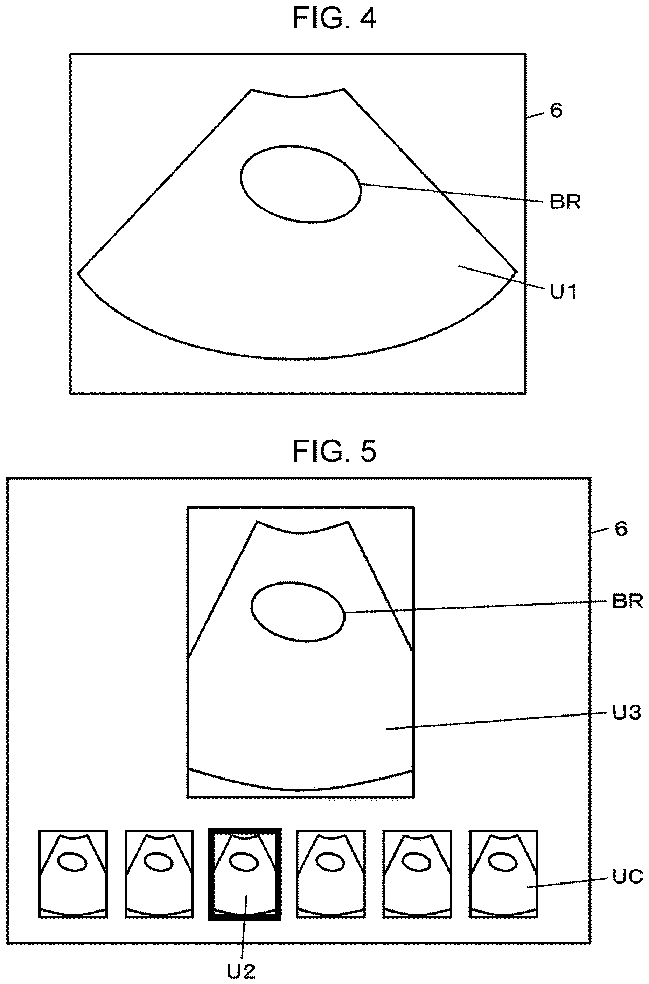

For example, as illustrated in Fig. 4, the urinary bladder extraction unit 9 extracts a urinary bladder region BR from an ultrasound image U1. For example, the urinary bladder extraction unit 9 may use a technique using deep learning described in Krizhevsk et al.: ImageNet Classification with Deep Convolutional Neural Networks, Advances in Neural Information Processing Systems 25, pp. 1106-1114 (2012) to extract the urinary bladder region BR in the ultrasound image U1. Alternatively, the urinary bladder extraction unit 9 may use, as another technique, a known technique such as graph cut (Y. Boykov and V. Kolmogorov, "An experimental comparison of min-cut/max-flow algorithm for energy minimization in vision", IEEE Transactions on Pattern Analysis and Machine Intelligence, 26, 9, pp. 1123-1137, 2004.), Snakes (A. W. Michael Kass and D. Terzopoulos: "Snakes: Active contour models", Int. J. Computer Vision, 1, 4, pp. 321-331, 1988.), or Level Sets (M. Sussman, P. Smereka, and S. Osher: "A level set approach for computing solutions to incompressible two-phase flow", J. Comput. Phys, 114, 1, pp. 146-159, 1994) to extract the urinary bladder region BR.

The feature quantity calculation unit 10 calculates a feature quantity related to the extracted urinary bladder region BR in the ultrasound image U1 from which the urinary bladder region BR has been extracted by the urinary bladder extraction unit 9. Through image analysis, the feature quantity calculation unit 10 can calculate, as the feature quantity, an area of the extracted urinary bladder region BR, for example. Through image analysis, the feature quantity calculation unit 10 can calculate, as the feature quantity, the largest diameters of the urinary bladder region BR in three directions orthogonal to each other, which are used for measuring the volume of the urinary bladder described below, for example. Through image analysis, the feature quantity calculation unit 10 can calculate, as the feature quantities, the largest diameter in any direction of the extracted urinary bladder region BR, the circumferential length of the urinary bladder region BR, and the like.

Based on the feature quantity calculated by the feature quantity calculation unit 10 for each of the ultrasound images of the second frame group formed by the frame thinning-out unit 8, the candidate frame extraction unit 11 extracts an ultrasound image of at least one candidate frame that serves as a measurement candidate for urine volume measurement of the subject from the ultrasound images U1 of the second frame group. For example, the candidate frame extraction unit 11 can extract, as the ultrasound images of the candidate frames, ultrasound images of a certain number of frames in order from the frame having the largest feature quantity calculated by the feature quantity calculation unit 10 from the ultrasound images of the second frame group.

For example, as illustrated in Fig. 5, the candidate frame extraction unit 11 causes the monitor 6 to display an ultrasound image UC of at least one extracted candidate frame. In the example illustrated in Fig. 5, ultrasound images UC of six candidate frames are displayed in a lower portion of the monitor 6. When the ultrasound images UC of the plurality of candidate frames are extracted, the candidate frame extraction unit 11 can display the ultrasound images UC of all the extracted candidate frames together on the monitor 6. The candidate frame extraction unit 11 can also display only some of the ultrasound images UC of all the extracted candidate frames together on the monitor 6 in so-called scroll display.

The input device 15 is a device with which a user performs an input operation, and can include a keyboard, a mouse, a trackball, a touch pad, a touch panel, and the like.

The measurement frame selection unit 12 selects, as a measurement frame that serves as a target subjected to measurement, an ultrasound image of a frame selected by the user via the input device 15 from the ultrasound image UC of the at least one candidate frame displayed on the monitor 6. For example, in Fig. 5, an ultrasound image U2 of a frame selected by the user from the ultrasound images UC of the plurality of candidate frames displayed in the lower portion of the monitor 6 is displayed with a thick frame, and the ultrasound image U2 of this frame is selected as the ultrasound image of the measurement frame by the measurement frame selection unit 12.

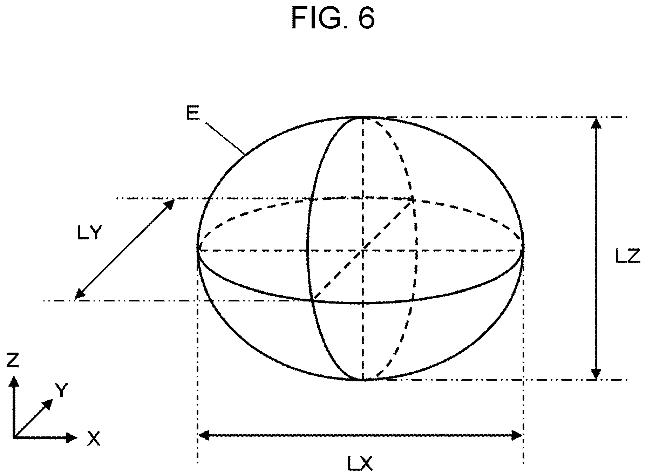

The urine volume measurement unit 13 calculates the volume of the urinary bladder of the subject based on the ultrasound image of the measurement frame selected by the measurement frame selection unit 12 to measure the urine volume in the urinary bladder. Since the urinary bladder generally has a substantially ellipsoidal shape, the urine volume measurement unit 13 calculates the volume of the urinary bladder as the volume of the ellipsoid. As illustrated in Fig. 6, when an ellipsoid E has a symmetrical shape with respect to an XY plane, an YZ plane, an XZ plane, it is known that the volume of the ellipsoid E is calculated by (LX × LY × LZ) × π/6, where LX is the largest diameter of the ellipsoid E in the X direction, LY is the largest diameter in the Y direction, LZ is the largest diameter in the Z direction, and π is the circumference ratio. Thus, when the volume of the urinary bladder is calculated using an ultrasound image, it is desirable to perform measurement on ultrasound images of two frames corresponding to scan cross-sections that pass through the center of the urinary bladder and are orthogonal to each other.

For example, when ultrasound images of two measurement frames corresponding to scan cross-sections that pass through the center of the urinary bladder and are orthogonal to each other are selected by the measurement frame selection unit 12, the urine volume measurement unit 13 measures lengths of the urinary bladder region BR in the ultrasound images of the two measurement frames, acquires the largest diameters LX, LY, and LZ in three directions orthogonal to each other, and calculate (LX × LY × LZ) × π/6 to calculate the volume of the urinary bladder of the subject.

Under the control of the device control unit 14, the display control unit 5 performs predetermined processing on the ultrasound images of the first frame group stored in the image memory 7, the ultrasound image UC of at least one candidate frame extracted by the candidate frame extraction unit 11, and information indicating the value of urine volume in the urinary bladder of the subject measured by the urine volume measurement unit 13, and displays them on the monitor 6.

Under the control of the display control unit 5, the monitor 6 displays the ultrasound images of the first frame group, the ultrasound image UC of the at least one candidate frame, the value of the urine volume in the urinary bladder of the subject, and the like. The monitor 6 includes a display device such as a liquid crystal display (LCD) or an organic electroluminescence (EL) display, for example.

The device control unit 14 controls each unit of the ultrasound diagnostic apparatus 1, based on a control program or the like stored in advance.

The processor 22 having the image generation unit 4, the display control unit 5, the frame thinning-out unit 8, the urinary bladder extraction unit 9, the feature quantity calculation unit 10, the candidate frame extraction unit 11, the measurement frame selection unit 12, the urine volume measurement unit 13, and the device control unit 14 may be constituted by a central processing unit (CPU) and a control program for causing the CPU to perform various processes. However, the processor 22 may be constituted using a field programmable gate array (FPGA), a digital signal processor (DSP), an application specific integrated circuit (ASIC), a graphics processing unit (GPU), another integrated circuit (IC), or any combination thereof.

The image generation unit 4, the display control unit 5, the frame thinning-out unit 8, the urinary bladder extraction unit 9, the feature quantity calculation unit 10, the candidate frame extraction unit 11, the measurement frame selection unit 12, the urine volume measurement unit 13, and the device control unit 14 of the processor 22 may be partially or entirely integrated into a single CPU or the like.

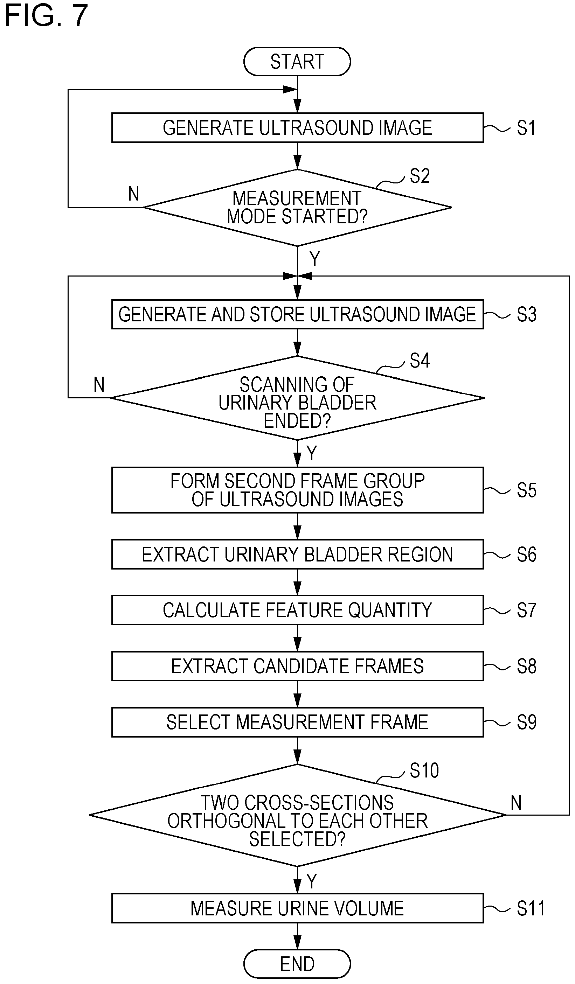

An operation of the ultrasound diagnostic apparatus 1 according to the first embodiment will be described in detail below with reference to a flowchart illustrated in Fig. 7. First, in step S1, an ultrasound image is generated with the ultrasound probe 21 being kept in contact with a body surface of the subject by the user, and the generated ultrasound image is displayed on the monitor 6. At this time, an ultrasonic beam is transmitted from the plurality of transducers of the transducer array 2 into the subject in accordance with drive signals from the pulser 23 of the transmission/reception circuit 3. The transducers receive an ultrasound echo from the subject and output respective reception signals to the amplifier 24 of the transmission/reception circuit 3. The reception signals are amplified by the amplifier 24, are subjected to AD conversion by the AD converter 25, and are then subjected to phasing addition by the beamformer 26, so that a sound ray signal is generated. In the image generation unit 4, this sound ray signal is subjected to envelope detection processing by the signal processing unit 27 to be a B-mode image signal, and the B-mode image signal is output to the display control unit 5 through the DSC 28 and the image processing unit 29. Consequently, the ultrasound image U1 is displayed on the monitor 6 under the control of the display control unit 5 as illustrated in Fig. 4.

At this time, the user adjusts the position and the inclination of the ultrasound probe 21 while checking the ultrasound image U1 displayed on the monitor 6 so that the urinary bladder region BR of the subject is shown in the ultrasound image U1.

Next, in step S2, a determination is made as to whether or not a measurement mode for measuring the urine volume in the urinary bladder of the subject is started. For example, when an instruction for starting the measurement mode is given by the user via the input device 15, it is determined that the measurement mode is started. When an instruction for starting the measurement mode is not given by the user, it is determined that the measurement mode is not started. If it is determined that the measurement mode is not started, the process returns to step S1, in which the ultrasound image U1 is generated and displayed. If it is determined that the measurement mode is started in response to the user giving an instruction for starting the measurement mode after adjusting the position of the ultrasound probe 21, the process proceeds to step S3.

If it is determined in step S2 that the measurement mode is started, an ultrasound image is generated and the generated ultrasound image is stored in the image memory 7 in step S3 in substantially the same manner as in step S1. The user images the urinary bladder of the subject while changing the position or the inclination of the ultrasound probe 21 to scan the urinary bladder.

In subsequent step S4, it is determined whether or not scanning of the urinary bladder of the subject is ended. For example, if an instruction for ending scanning of the urinary bladder is given by the user via the input device 15, it is determined that scanning of the urinary bladder is ended. If an instruction for ending scanning of the urinary bladder is not given by the user, it is determined that scanning of the urinary bladder continues. If it is determined that scanning of the urinary bladder continues, the process returns to step S3, in which an ultrasound image is generated and stored. In this manner, steps S3 and S4 are repeated while scanning of the urinary bladder continues. In this manner, ultrasound images of the first frame group constituted by ultrasound images of a plurality of frames is stored in the image memory 7. If it is determined that scanning of the urinary bladder is ended, the process proceeds to step S5.

In step S5, the frame thinning-out unit 8 extracts an ultrasound image at predetermined intervals in time series from the ultrasound images of the first frame group stored in the image memory 7, and removes the ultrasound images of the rest of the frames that are not extracted. In this manner, the frame thinning-out unit 8 forms the second frame group of ultrasound images. At this time, the frame thinning-out unit 8 can extract an ultrasound image at intervals of predetermined frames such as five to ten frames in time series from the ultrasound images of the first frame group, for example. The frame thinning-out unit 8 can extract an ultrasound image at predetermined time intervals in time series from the ultrasound images of the first frame group, for example.

In general, among the ultrasound images of a plurality of frames consecutively generated, the ultrasound images of the frames adjacent to each other in time series are imaged at adjacent times. Thus, a change is often small between the images. In step S5, since the ultrasound images are extracted at predetermined intervals in time series from the ultrasound images of the first frame group, the second frame group of ultrasound images can be formed from the ultrasound images of the frames not adjacent to each other in time series to include many ultrasound images of frames that are dissimilar from each other.

Through the processing of step S5, the second frame group of ultrasound images constituted by ultrasound images of a smaller number of frames than the ultrasound images of the first frame group is formed. Thus, an amount of calculation performed by the ultrasound diagnostic apparatus 1 at the time of extraction of the ultrasound images UC of the candidate frames that serve as candidates subjected to urine volume measurement in the urinary bladder can be reduced, and the ultrasound images UC of the candidate frames can be extracted more quickly.

Subsequently, in step S6, the urinary bladder extraction unit 9 performs image analysis on each of the ultrasound images of the second frame group formed in step S5, and extracts a urinary bladder region BR representing the urinary bladder of the subject. Information of the extracted urinary bladder region BR and the ultrasound images of the second frame group are sent to the feature quantity calculation unit 10.

In step S7, based on the urinary bladder region BR extracted in step S6 from each of the ultrasound images of the second frame group, the feature quantity calculation unit 10 calculates feature quantities related to the urinary bladder region BR, such as the area of the urinary bladder region BR and the largest diameters of the urinary bladder region BR as illustrated in Fig. 4. Information of the calculated feature quantities and the ultrasound images of the second frame group are sent to the candidate frame extraction unit 11.

In subsequent step S8, based on the feature quantities calculated in step S7 for the respective ultrasound images of the second frame group, the candidate frame extraction unit 11 extracts an ultrasound image UC of at least one candidate frame, which serves as a candidate subjected to urine volume measurement in the urinary bladder of the subject from the ultrasound images of the second frame group. For example, the candidate frame extraction unit 11 extracts, as the ultrasound images UC of the candidate frames, ultrasound images of a certain number of frames in order from the ultrasound image of the frame with the largest feature quantity calculated in step S7.

The candidate frame extraction unit 11 displays the extracted ultrasound image UC of the at least one candidate frame on the monitor 6 as illustrated in Fig. 5, for example. In the example of Fig. 5, the ultrasound images UC of six candidate frames are displayed in the lower portion of the monitor 6.

In response to the ultrasound image UC of the at least one candidate frame being displayed on the monitor 6 in step S8, the ultrasound image U2 of one frame is selected from the ultrasound image UC of the at least one candidate frame displayed on the monitor 6 by the user via the input device 15 in step S9 as illustrated in Fig. 5. The ultrasound image U2 of the frame selected by the user is selected as the ultrasound image of the measurement frame by the measurement frame selection unit 12. In Fig. 5, the ultrasound image U2 of the frame indicated by a thick frame is selected by the user and is selected as the measurement frame by the measurement frame selection unit 12. At this time, for example, an ultrasound image U3 obtained by enlarging the ultrasound image U2 of the selected frame can be displayed on the monitor 6 to allow the user to easily check the ultrasound image U2 of the selected frame.

In this manner, the ultrasound image U2 of the frame selected by the user is selected as the ultrasound image of the measurement frame from the ultrasound image UC of the at least one candidate frame displayed on the monitor 6. Thus, even if there is a frame in which the urinary bladder region BR is erroneously extracted in step S6, the ultrasound image of the measurement frame can be selected from the frames in which the urinary bladder region BR is correctly extracted. Consequently, the accuracy of urine volume measurement can be increased. The ultrasound image UC of the at least one candidate frame is further extracted from the ultrasound images of the second frame group that are less than the ultrasound images of the first frame group, the user need not check ultrasound images of many frames. Consequently, the load imposed on the user in selecting the ultrasound image of the measurement frame can be reduced.

In step S10, it is determined whether or not ultrasound images of two measurement frames corresponding to two scan cross-sections of the urinary bladder of the subject orthogonal to each other have been selected in step S9 in order to measure the urine volume in the urinary bladder. In step S9 that has already been completed, only an ultrasound image of the measurement frame corresponding to one of the two scan cross-sections of the urinary bladder orthogonal to each other has been acquired. Thus, it is determined that ultrasound images of two measurement frames corresponding to the two scan cross-sections of the urinary bladder of the subject orthogonal to each other are not selected, the process returns to step S3, in which scanning of the urinary bladder is resumed. At this time, the user rotates the orientation of the ultrasound probe 21 by 90 degrees and scans the urinary bladder.

In steps S3 and S4, an ultrasound image is repeatedly generated and stored unless the user gives an instruction for ending scanning of the urinary bladder. In response to the user giving the instruction for ending scanning of the urinary bladder in step S4, the process proceeds to step S5. Description of the subsequent processing of steps S5 to S9 is omitted since the details thereof are identical to those already described.

In step S9, the ultrasound image U2 of the frame selected by the user is selected as the ultrasound image of the measurement frame from the ultrasound image UC of the at least one candidate frame displayed on the monitor 6. The process then proceeds to step S10.

In step S10, it is determined whether or not ultrasound images of two measurement frames corresponding to two scan cross-sections of the urinary bladder of the subject orthogonal to each other have been selected in step S9. In step S9 performed for the second time, the ultrasound image of the second measurement frame has been selected. Thus, it is determined that the ultrasound images of the two measurement frames corresponding to the two scan cross-sections of the urinary bladder of the subject orthogonal to each other are selected. The process then proceeds to step S11.

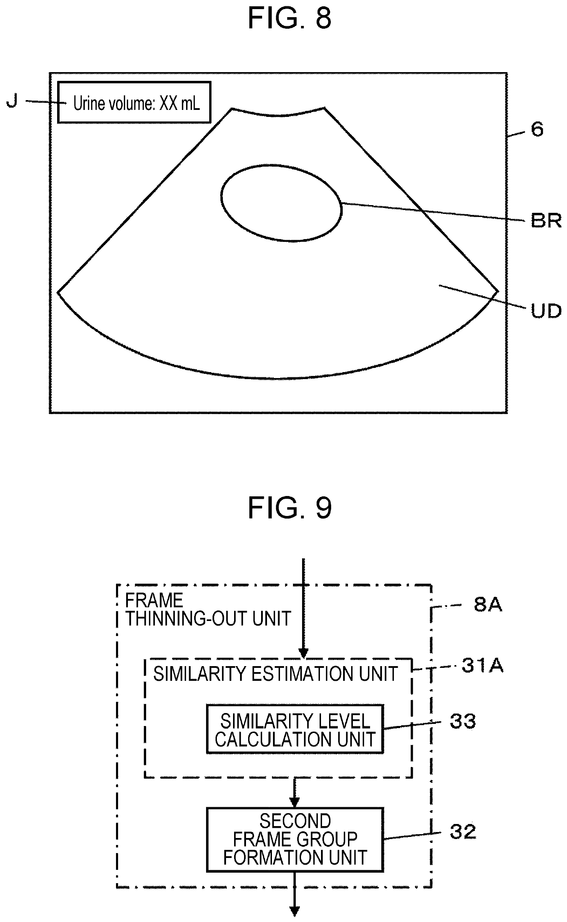

In step S11, the urine volume measurement unit 13 extracts the urinary bladder region BR from each of the ultrasound images of the two measurement frames selected by the user in step S9 performed twice and calculates the volume of the urinary bladder of the subject based on the diameters of the extracted urinary bladder region BR to measure the urine volume in the urinary bladder. For example, the urine volume measurement unit 13 regards the urinary bladder as the ellipsoid E as illustrated in Fig. 6, measures the largest diameters LX, LY, and LZ respectively in the X, Y, and Z directions of the ellipsoid E from the ultrasound images of the two measurement frames selected by the user in step S9, and calculates (LX × LY × LZ) × π/6. In this manner, the urine volume measurement unit 13 can calculate the volume of the ellipsoid E as the volume of the urinary bladder. For example, as illustrated in Fig. 8, the urine volume measurement unit 13 displays a measured urine volume J in the urinary bladder on the monitor 6. In the example of Fig. 8, the ultrasound image UD of the measurement frame selected by the user in the step S9 performed for the second time and the urine volume J in the urinary bladder are displayed together on the monitor 6.

In response to the urine volume J in the urinary bladder of the subject being measured in this manner, the operation of the ultrasound diagnostic apparatus 1 illustrated in the flowchart of Fig. 7 ends.

As described above, the ultrasound diagnostic apparatus according to the first embodiment of the present invention forms a second frame group of ultrasound images by thinning out ultrasound images of some frames from ultrasound images of a first frame group, further extracts an ultrasound image UC of at least one candidate frame from the ultrasound images of the second frame group, and selects the ultrasound image U2 of the frame selected by the user as the ultrasound image UD of the measurement frame from the ultrasound image UC of the at least one candidate frame displayed on the monitor 6. Thus, when selecting the ultrasound image UD of the measurement frame, the user need not check ultrasound images of many frames, and the load imposed on the user can be reduced.

The ultrasound images of the frames similar to each other are less in the ultrasound images of the second frame group than in the ultrasound images of the first frame group. Thus, even if the ultrasound image UC of the at least one candidate frame extracted from the ultrasound images of the second frame group includes an ultrasound image of a frame in which the urinary bladder region BR is erroneously extracted, the ultrasound diagnostic apparatus 1 according to the first embodiment of the present invention allows the ultrasound image UD of the suitable measurement frame to be easily selected from the ultrasound images of the candidate frames in which the urinary bladder region BR is correctly extracted. Consequently, the accuracy of urine volume measurement can be increased.

The beamformer 26 that performs so-called reception focusing processing is included in the transmission/reception circuit 3. However, the beamformer 26 may be included in the image generation unit 4, for example. Even in this case, as in the case where the beamformer 26 is included in the transmission/reception circuitry 3, an ultrasound image is generated by the image generation unit 4.

The image generation unit 4 is included in the processor 22. However, the image generation unit 4 may be included in the ultrasound probe 21.

It is determined in step S4 that scanning of the urinary bladder is ended if an instruction for ending scanning of the urinary bladder is given by the user. However, for example, it may be determined that scanning of the urinary bladder is ended when a certain time period such as 15 seconds, for example, has elapsed from a timing at which the measurement mode is started in step S2 and the generation and storage of the ultrasound image are started in step S3. This case saves the user time of giving an instruction for ending scanning of the urinary bladder.

For example, it may be determined whether or not the ultrasound probe 21 is in contact with the body surface of the subject, and control may be performed to start and end scanning of the urinary bladder in accordance with the determined result. When the ultrasound probe 21 is in contact with the body surface of the subject, an ultrasound image of a frame corresponding to a scan cross-section of the subject is generated. By contrast, when the ultrasound probe 21 is separate from the subject to be in a so-called aerial radiation state, an entirely black ultrasound image is usually generated. Thus, for example, by analyzing the generated ultrasound image, it can be determined whether or not the ultrasound probe 21 is in contact with the body surface of the subject. Accordingly, for example, when it is determined that the ultrasound probe 21 is in contact with the body surface of the subject, scanning of the urinary bladder can be started, and when it is determined that the ultrasound probe 21 is separate from the body surface of the subject, scanning of the urinary bladder can be ended. This case also saves the user time of giving an instruction for ending scanning of the urinary bladder.

The above-described plurality of methods for determining the start and end of scanning of the urinary bladder can be appropriately combined with each other.

After all the ultrasound images of the first frame group are stored in the image memory 7, the processing of extracting the ultrasound image is performed on all the ultrasound images of the first frame group in step S5. However, the processing of extracting the ultrasound image may be sequentially performed on the ultrasound images of the frames stored in the image memory 7 while scanning of the urinary bladder is determined to be continued in step S4. In this case, for example, from ultrasound images of frames that are consecutively generated and stored in the image memory 7 through repetitions of steps S3 and S4, an ultrasound image is extracted at predetermined intervals in time series, and ultrasound images of frames that are not extracted are removed.

In this manner, the ultrasound images are extracted in real time from the ultrasound images of the first frame group, so that the time from the start of generation of the ultrasound image in step S3 to the end of formation of the second frame group of ultrasound images can be reduced. Since the processing of steps S6 and S7 can be sequentially performed on the ultrasound images of the frames extracted in real time from the ultrasound images of the first frame group, the waiting time can be reduced and the candidate frames can be extracted more quickly.

In step S8, the method of extracting the ultrasound image UC of the at least one candidate frame from the ultrasound images of the second frame group is not limited particularly to the method of extracting ultrasound images of a certain number of frames in order from the ultrasound image of the frame with the largest feature quantity among the ultrasound images of the second frame group.

For example, the candidate frame extraction unit 11 can extract, as the ultrasound image UC of the candidate frame, an ultrasound image of a frame for which the feature quantity calculated in step S7 is greater than or equal to a predetermined feature quantity threshold. The feature quantity threshold can be set in advance by the user, for example. For example, the feature quantity threshold used may be a value obtained by multiplying the largest value of the feature quantity calculated in step S7 by a predetermined ratio.

For example, in the case where an ultrasound image is extracted in real time from the ultrasound images of the first frame group, a feature quantity may be calculated for the extracted ultrasound image of the frame whenever necessary, and it may be determined whether or not the calculated feature quantity is greater than or equal to the predetermined feature quantity threshold. In this manner, the ultrasound image of the candidate frame can be extracted in real time.

For example, the candidate frame extraction unit 11 may sequentially store, as the ultrasound image UC of the candidate frame, the ultrasound image of the frame extracted in real time from the ultrasound images of the first frame group, and remove the ultrasound image in order from the frame with the smallest feature quantity from the stored ultrasound images of the frames when the number of frames of the stored ultrasound images exceeds a predetermined number. In this manner, the candidate frame extraction unit 11 can update the ultrasound images UC of the candidate frames.

When the ultrasound images UC of the plurality of candidate frames are extracted in step S8, the extracted ultrasound images UC of the candidate frames can be displayed on the monitor 6 in chronological order. For example, as illustrated in Fig. 5, in the case where the ultrasound images UC of the plurality of candidate frames are displayed adjacently to each other in the horizontal direction of the monitor 6, the ultrasound images UC of the plurality of candidate frames can be displayed such that the ultrasound image UC displayed on the left side is the ultrasound image UC generated at an earlier time and the ultrasound image UC displayed on the right side is the ultrasound image generated at a later time.

For example, the ultrasound images UC of the plurality of candidate frames may be displayed in descending order of the feature quantity.

If the ultrasound images UC of the plurality of candidate frames are rearranged based on a specific criterion and the rearranged ultrasound images UC are displayed on the monitor 6 in this manner, the user can more easily check the ultrasound images UC of the plurality of candidate frames and select the ultrasound image U2 of the one frame.

When the ultrasound image UC of the at least one candidate frame is displayed on the monitor 6 in step S8, the feature quantity calculated in step S7 may be displayed together with the ultrasound image UC of the at least one candidate frame. In this case, the user can select the ultrasound image U2 of the one frame from the ultrasound image UC of the at least one candidate frame with reference to the value of the feature quantity displayed on the monitor 6. Thus, the user can more easily select the ultrasound image UD of the measurement frame suitable for urine volume measurement. Consequently, the accuracy of urine volume measurement can be increased.

The operation of the ultrasound diagnostic apparatus 1 returns to step S3 if it is determined in step S10 that ultrasound images of two measurement frames corresponding to two scan cross-sections of the urinary bladder of the subject orthogonal to each other are not selected. For example, immediately after step S10, a message prompting rotation of the orientation of the ultrasound probe 21 by 90° may be displayed on the monitor 6. If an instruction regarding an operation to be performed on the ultrasound probe 21 is given to the user in this manner, the user can more smoothly proceed with the procedure of urine volume measurement.

The frame thinning-out unit 8 can calculate the processing speed of the ultrasound diagnostic apparatus 1 by acquiring an operating frequency of the CPU and the like of the processor 22. Based on the calculated processing speed, the frame thinning-out unit 8 can update the predetermined intervals at which the ultrasound images are extracted from the ultrasound images of the first frame group in step S5. For example, the frame thinning-out unit 8 has a processing speed threshold for the processing speed of the ultrasound diagnostic apparatus 1. If the calculated processing speed is lower than or equal to the processing speed threshold, the frame thinning-out unit 8 can increase the predetermined intervals to reduce the frequency with which the ultrasound images are extracted from the ultrasound images of the first frame group. If the calculated processing speed is higher than the processing speed threshold, the frame thinning-out unit 8 can reduce the predetermined intervals to increase the frequency with which ultrasound images are extracted from the ultrasound images of the first frame group. Thus, even when the processing speed of the ultrasound diagnostic apparatus 1 is low, the ultrasound diagnostic apparatus 1 can form the second frame group of ultrasound images while reducing the influence of the processing speed.

The image memory 7 stores the ultrasound images of the frames generated by the image generation unit 4 as the ultrasound images of the first frame group. The image memory 7 can also store ultrasound images of a series of frames input from the outside, as the ultrasound images of the first frame group. In this case, urine volume measurement can be performed based on the ultrasound images of the plurality of frames input from the outside instead of the ultrasound images of the frames generated by the image generation unit 4. Thus, in this case, the ultrasound diagnostic apparatus 1 need not include the ultrasound probe 21 including the transducer array 2 and the transmission/reception circuit 3, and the image generation unit 4.

### Second Embodiment

In the first embodiment, the frame thinning-out unit 8 forms the second frame group of ultrasound images by extracting ultrasound images at predetermined intervals in time series from the ultrasound images of the first frame group. However, the method of forming the second frame group of ultrasound images is not limited this. For example, the second frame group of ultrasound images can be formed by extracting ultrasound images from the ultrasound images of the first frame group, based on similarity levels between the ultrasound images of the frames different from each other in the first frame group.

An ultrasound diagnostic apparatus according to the second embodiment is equivalent to the ultrasound diagnostic apparatus 1 according to the first embodiment illustrated in Fig. 1 that includes a frame thinning-out unit 8A illustrated in Fig. 9 instead of the frame thinning-out unit 8. The frame thinning-out unit 8A includes a similarity estimation unit 31A and a second frame group formation unit 32. The similarity estimation unit 31A includes a similarity level calculation unit 33. The second frame group formation unit 32 is connected to the similarity estimation unit 31A.

The similarity level calculation unit 33 performs so-called matching processing or the like on ultrasound images of two frames different from each other to calculate a similarity level between the ultrasound images of the two frames different from each other.

The similarity estimation unit 31A sets an ultrasound image of a reference frame serving as a reference of similarity estimation and an ultrasound image of an estimation-target frame serving as a target subjected to similarity estimation from the ultrasound images of the first frame group. The similarity estimation unit 31A calculates a similarity level between the set ultrasound image of the reference frame and the set ultrasound image of the estimation-target frame by using the similarity level calculation unit 33. Based on the calculated similarity level, the similarity estimation unit 31A estimates whether or not the ultrasound image of the estimation-target frame is similar to the ultrasound image of the reference frame.

The similarity estimation unit 31A has, for example, a predetermined similarity level threshold for the similarity level calculated by the similarity level calculation unit 33. If the similarity level calculated by the similarity level calculation unit 33 is greater than or equal to the predetermined similarity level threshold, the similarity estimation unit 31A estimates that the ultrasound image of the estimation-target frame is similar to the ultrasound image of the reference frame. If the similarity level calculated by the similarity level calculation unit 33 is less than the similarity level threshold, the similarity estimation unit 31A estimates that the ultrasound image of the estimation-target frame is dissimilar from the ultrasound image of the reference frame.

The second frame group formation unit 32 removes the ultrasound image of the estimation-target frame estimated to be similar to the ultrasound image of the reference frame by the similarity estimation unit 31A from the ultrasound images of the first frame group, and forms the second frame group of ultrasound images using the ultrasound images of the rest of the frames.

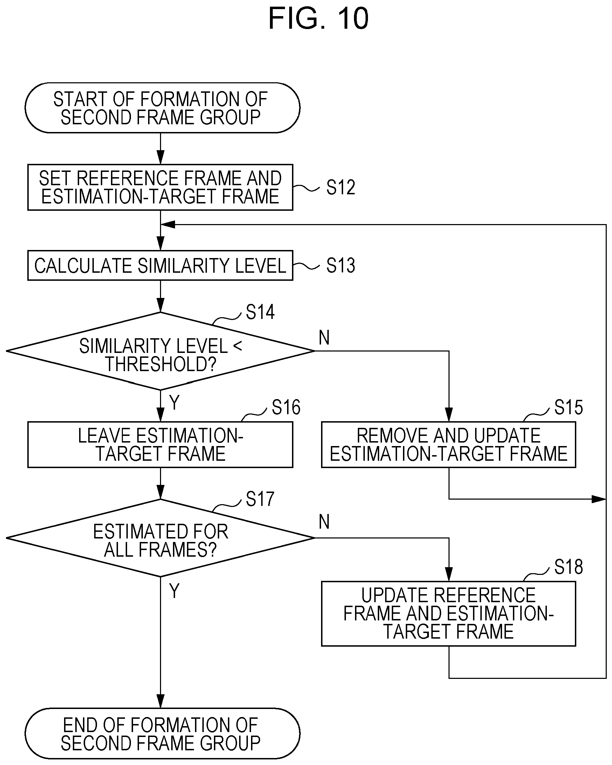

An operation of the ultrasound diagnostic apparatus according to the second embodiment will be described next. The operation of the ultrasound diagnostic apparatus according to the second embodiment is equivalent to the operation of the ultrasound diagnostic apparatus 1 according to the first embodiment illustrated in Fig. 7 in which the second frame group of ultrasound images is formed through a process illustrated in a flowchart of Fig. 10 instead of the processing of step S5 of extracting ultrasound images at predetermined intervals in time series from the ultrasound images of the first frame group and forming the second frame group of ultrasound images. An operation of forming the second frame group of ultrasound images in the second embodiment will be described below with reference to the flowchart of Fig. 10.

First, in step S12, the similarity estimation unit 31A sets, as the ultrasound image of the reference frame, an ultrasound image of a frame generated at the earliest timing among the ultrasound images of the first frame group stored in the image memory 7, and sets, as the ultrasound image of the estimation-target frame, an ultrasound image of a frame generated subsequently to the ultrasound image of the reference frame in time series.

In step S13, the similarity level calculation unit 33 calculates the similarity level between the ultrasound image of the reference frame and the ultrasound image of the estimation-target frame by performing so-called matching processing or the like on the ultrasound image of the reference frame and the ultrasound image of the estimation-target frame set in step S12.

In step S14, the similarity estimation unit 31A determines whether or not the similarity level calculated in step S13 is less than the predetermined similarity level threshold to estimate whether or not the ultrasound image of the estimation-target frame set in step S12 is similar to the ultrasound image of the reference frame. If the similarity level calculated in step S13 is greater than or equal to the predetermined similarity level threshold, the similarity estimation unit 31A estimates that the ultrasound image of the estimation-target frame is similar to the ultrasound image of the reference frame. In this case, the process proceeds to step S15.

In step S15, the second frame group formation unit 32 removes the ultrasound image of the estimation-target frame estimated to be similar to the ultrasound image of the reference frame in step S14, and the similarity estimation unit 31A updates the ultrasound image of the estimation-target frame to an ultrasound image of the frame generated subsequently to the removed ultrasound image of the frame.

In response to completion of the processing of step S15, the process returns to step S13, in which the similarity level calculation unit 33 calculates the similarity level between the ultrasound image of the reference frame and the ultrasound image of the new estimation-target frame updated in step S15. While the similarity level calculated in step S13 is greater than or equal to the predetermined similarity level threshold and the ultrasound image of the estimation-target frame is estimated to be similar to the ultrasound image of the reference frame, removal and updating of the ultrasound image of the estimation-target frame are repeated through repetition of the processing of steps S13 to S15.

If it is determined in step S14 that the similarity calculated in step S13 is less than the predetermined similarity level threshold and the ultrasound image of the estimation-target frame is estimated to be dissimilar to the ultrasound image of the reference frame, the process proceeds to step S16.

In step S16, the second frame group formation unit 32 leaves the ultrasound image of the estimation-target frame estimated to be dissimilar to the ultrasound image of the reference frame in step S14 as the ultrasound image that constitutes the second frame group.

In subsequent step S17, it is determined whether or not all the ultrasound images of the first frame group stored in the image memory 7 have been subjected to similarity estimation by the similarity estimation unit 31A. If it is determined that all the ultrasound images of the first frame group have not been subjected to similarity estimation, the process proceeds to step S18.

In step S18, the similarity estimation unit 31A updates the ultrasound image of the reference frame to the ultrasound image of the estimation-target frame left in step S16, and updates the ultrasound image of the estimation-target frame to an ultrasound image of the frame generated subsequently to the updated ultrasound image of the reference frame. In response to completion of the processing of step S18 in this way, the process returns to step S13, in which the similarity level calculation unit 33 calculates the similarity level between the ultrasound image of the new reference frame updated in step S18 and the ultrasound image of the new estimation-target frame updated in step S18.

While it is determined in step S17 that the similarity estimation has not been performed on all the ultrasound images of the first frame group, steps S13 to S18 are repeated, so that ultrasound images of frames estimated to be similar to the ultrasound image of the reference frame are removed from the ultrasound images of the first frame group and ultrasound images of frames estimated to be dissimilar to the ultrasound image of the reference frame are left.

If it is determined in step S17 that the similarity estimation has been performed on all the ultrasound images of the first frame group, the second frame group formation unit 32 forms the second frame group of ultrasound images using the all the ultrasound images of the frames left in step S16.

Then, the operation of forming the second frame group of ultrasound images in the second embodiment ends.

As described above, the ultrasound diagnostic apparatus according to the second embodiment calculates a similarity level between ultrasound images of two frames different from each other among the ultrasound images of the first frame group, and removes, based on the calculated similarity level, the ultrasound image from the ultrasound images of the first frame group. In this manner, the ultrasound diagnostic apparatus according to the second embodiment can form the second frame group of ultrasound images constituted by ultrasound images of a smaller number of frames than the number of frames of the ultrasound images of the first frame group by thinning out ultrasound images of some frames from the ultrasound images of the first embodiment as in the processing of step S5 in the first embodiment. Thus, when selecting the ultrasound image UD of the measurement frame, the user need not check ultrasound images of many frames. Consequently, the load imposed on the user can be reduced.

In the second embodiment, since the second frame group of ultrasound images is constituted by ultrasound images of frames dissimilar from each other, the user can easily select the ultrasound image UD of the suitable measurement frame from the ultrasound images of the candidate frames in which the urinary bladder region BR is correctly extracted. Thus, the ultrasound diagnostic apparatus according to the second embodiment can also increase the accuracy of urine volume measurement.

### Third Embodiment

In the second embodiment, the similarity level between the ultrasound images of the two frames different from each other among the ultrasound images of the first frame group is calculated, and the second frame group of ultrasound images is formed based on the calculated similarity level. Instead of the similarity level, an area ratio between the urinary bladder regions BR in the respective ultrasound images of the two frames different from each other may be used.

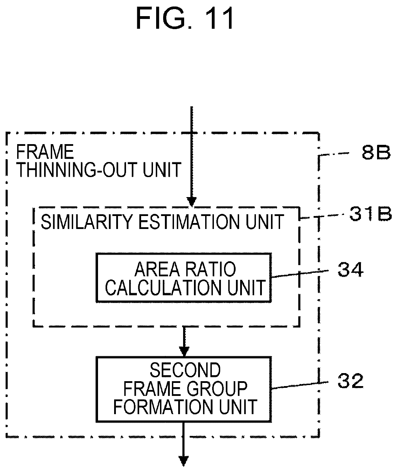

An ultrasound diagnostic apparatus according to a third embodiment is equivalent to the ultrasound diagnostic apparatus according to the second embodiment that includes a frame thinning-out unit 8B illustrated in Fig. 11 instead of the frame thinning-out unit 8A illustrated in Fig. 9. The frame thinning-out unit 8B is equivalent to the frame thinning-out unit 8A in the second embodiment that includes a similarity estimation unit 31B instead of the similarity estimation unit 31A. The similarity estimation unit 31B includes an area ratio calculation unit 34. The second frame group formation unit 32 is connected to the similarity estimation unit 31B.

The area ratio calculation unit 34 extracts the urinary bladder regions BR from the respective ultrasound images of the two frames different from each other among the ultrasound images of the first frame group, calculates through, for example, image analysis areas of the extracted urinary bladder regions BR, and calculate a ratio of values of the two calculated areas. In this manner, the area ratio calculation unit 34 calculates the area ratio between the urinary bladder regions BR in the respective ultrasound images of the two frames different from each other.

As the area ratio between the urinary bladder regions BR in the respective ultrasound images of the two frames different from each other becomes closer to 1, the values of the areas of the urinary bladder regions BR in the respective ultrasound images of the two frames become closer to each other. Thus, it is considered that the probability of the ultrasound images of the two frames being similar to each other increases. On the other hand, as the area ratio between the urinary bladder regions BR in the respective ultrasound images of the two frames different from each other is less or greater than 1, the difference between the areas of the urinary bladder regions BR in the respective ultrasound images of the two frames becomes greater. Thus, it is considered that the probability of the urinary bladder regions BR in the respective ultrasound images of the two frames being similar to each other decreases.

Based on such an idea, the similarity estimation unit 31B sets an ultrasound image of a reference frame and an ultrasound image of an estimation-target frame from the ultrasound images of the first frame group. The similarity estimation unit 31B calculates a ratio between an area of the urinary bladder region BR in the set ultrasound image of the reference frame and an area of the urinary bladder region BR in the set ultrasound image of the estimation-target frame by using the area ratio calculation unit 34. Based on the calculated area ratio, the similarity estimation unit 31B estimates whether or not the ultrasound image of the estimation-target frame is similar to the ultrasound image of the reference frame.

The similarity estimation unit 31B has, for example, a predetermined area ratio range including 1 such as a range greater than or equal to 0.8 and less than or equal to 1.2 for the area ratio calculated by the area ratio calculation unit 34. If the area ratio calculated by the area ratio calculation unit 34 is within the predetermined area ratio range, the similarity estimation unit 31B estimates that the ultrasound image of the estimation-target frame is similar to the ultrasound image of the reference frame. If the area ratio calculated by the area ratio calculation unit 34 is out of the predetermined area ratio range, the similarity estimation unit 31B estimates that the ultrasound image of the estimation-target frame is dissimilar from the ultrasound image of the reference frame.

Similarly to the second frame group formation unit 32 in the second embodiment, the second frame group formation unit 32 removes the ultrasound image of the estimation-target frame estimated to be similar to the ultrasound image of the reference frame by the similarity estimation unit 31B from the ultrasound images of the first frame group, and forms the second frame group of ultrasound images using the ultrasound images of the rest of the frames.

As described above, the ultrasound diagnostic apparatus according to the third embodiment calculates an area ratio between the urinary bladder regions BR in respective ultrasound images of two frames different from each other among the ultrasound images of the first frame group, and removes, based on the calculated area ratio, the ultrasound image from the ultrasound images of the first frame group. In this manner, the ultrasound diagnostic apparatus according to the third embodiment can form the second frame group of ultrasound images constituted by ultrasound images of a smaller number of frames than the number of frames of the ultrasound images of the first frame group as in the second embodiment. Thus, when selecting the ultrasound image UD of the measurement frame, the user need not check ultrasound images of many frames. Consequently, the load imposed on the user can be reduced.

Since the second frame group of ultrasound images is constituted by ultrasound images of frames dissimilar from each other, the user can easily select the ultrasound image UD of the suitable measurement frame from the ultrasound image UC of at least one candidate frame. Consequently, the accuracy of urine volume measurement can be increased.

The area ratio calculated by the area ratio calculation unit 34 by using the ultrasound image of the reference frame and the ultrasound image of the estimation-target frame may be an area ratio of the area of the urinary bladder region BR in the ultrasound image of the estimation-target frame to the area of the urinary bladder region BR in the ultrasound image of the reference frame or an area ratio of the area of the urinary bladder region BR in the ultrasound image of the reference frame to the area of the urinary bladder region BR in the ultrasound image of the estimation-target frame. Even when the area ratio is calculated using either the area of the urinary bladder region BR in the ultrasound image of the reference frame or the area of the urinary bladder region BR in the ultrasound image of the estimation-target frame as a denominator or a numerator, the similarity estimation unit 31B can estimate whether or not the ultrasound image of the estimation-target frame is similar to the ultrasound image of the reference frame depending on whether or not the calculated area ratio is within the predetermined area ratio range.

### Fourth Embodiment

An angle of the ultrasound probe 21 may be detected, and it may be determined, based on the detected angle, whether or not ultrasound images of two frames different from each other are similar to each other among the ultrasound images of the first frame group.

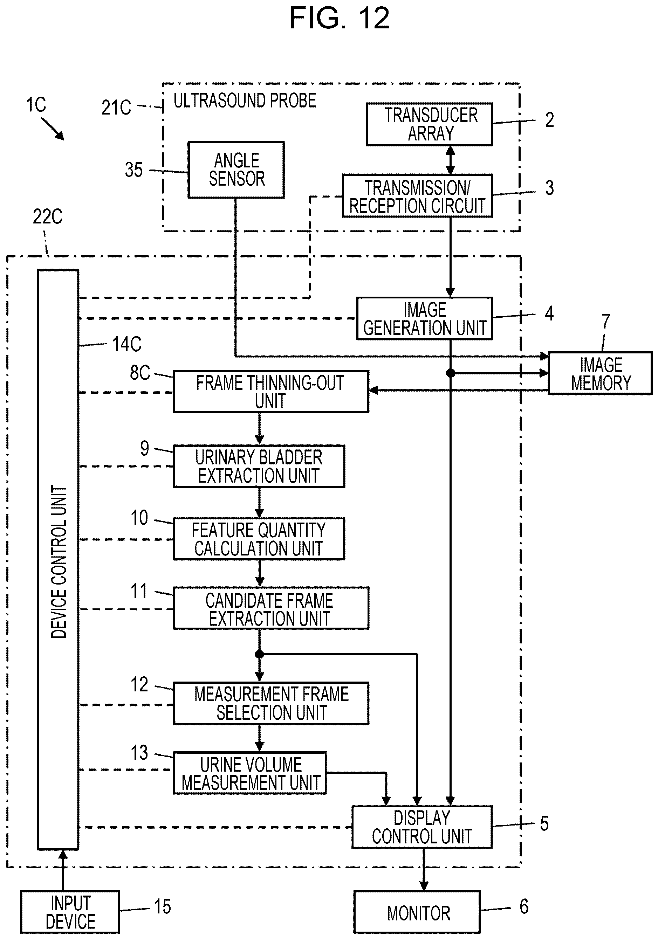

As illustrated in Fig. 12, an ultrasound diagnostic apparatus 1C according to a fourth embodiment is equivalent to the ultrasound diagnostic apparatus 1 according to the first embodiment illustrated in Fig. 1 that includes an ultrasound probe 21C instead of the ultrasound probe 21 and a processor 22C instead of the processor 22. The ultrasound probe 21C is equivalent to the ultrasound probe 21 in the first embodiment to which an angle sensor 35 is attached. The processor 22C is equivalent to the processor 22 in the first embodiment that includes a frame thinning-out unit 8C instead of the frame thinning-out unit 8 and a device control unit 14C instead of the device control unit 14.

The angle sensor 35 attached to the ultrasound probe 21C is connected to the image memory 7. The frame thinning-out unit 8C is connected to the image memory 7.

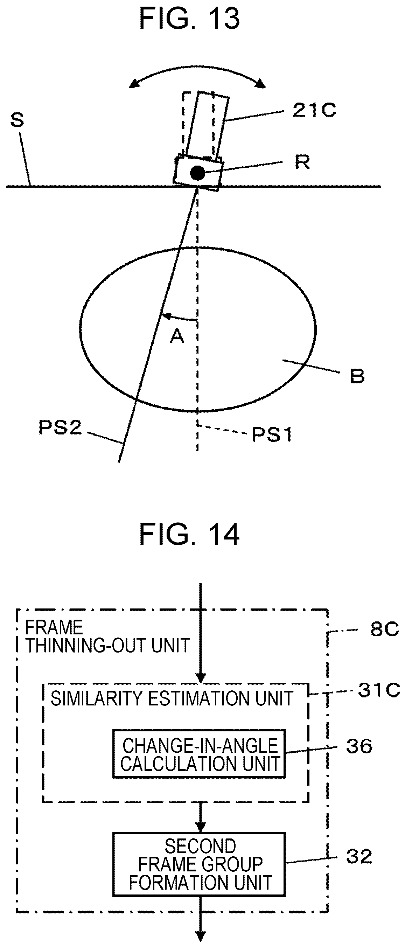

The angle sensor 35 is a sensor that detects an angle A of the ultrasound probe 21C illustrated in Fig. 13. The angle A refers to a rotation angle from a scan cross-section PS1 that is in a direction perpendicular to a body surface S of a subject to a scan cross-section PS2 in a state in which the ultrasound probe 21C is inclined, when the ultrasound probe 21C is inclined about a rotational axis R that is parallel to an arrangement direction of the transducer array 2 while a contact position of the ultrasound probe 21C with the body surface S of the subject is fixed. To distinguish an inclination direction of the ultrasound probe 21C with respect to a state where the angle A is 0, the angle sensor 35 can add a positive or negative sign to a magnitude of the detected angle A, for example. Fig. 13 illustrates an example in which the scan cross-sections PS1 and PS2 pass through the urinary bladder B of the subject.

The angle sensor 35 includes, for example, a so-called gyro sensor, an acceleration sensor, a magnetic sensor, or the like. The angle sensor 35 converts an electric signal obtained from the gyro sensor, the acceleration sensor, the magnetic sensor, or the like into the angle A of the ultrasound probe 21C by using a well-known calculation method or the like.

Information on the angle A of the ultrasound probe 21C detected in this way is sent to the image memory 7, and is stored in the image memory 7 together with an ultrasound image of a frame generated by the image generation unit 4 at the same time.

As illustrated in Fig. 14, the frame thinning-out unit 8C is configured such that a similarity estimation unit 31C and the second frame group formation unit 32 are connected in series. The similarity estimation unit 31C includes a change-in-angle calculation unit 36.

The change-in-angle calculation unit 36 calculates a difference in the angles A of the ultrasound probe 21C between ultrasound images of two frames different from each other among the ultrasound images of the first frame group stored in the image memory 7 to calculate a change in the angle of the ultrasound probe 21C.

When the ultrasound probe 21 is inclined about the rotational axis R while the contact position with the body surface S of the subject is fixed, it is considered that the smaller the change in the angle of the ultrasound probe 21C in a period from generation of one of the ultrasound images of the two frames different from each other to an end of generation of the other one, the closer the scan cross-sections represented by the ultrasound images of the two frames are to each other also in a deep portion of the subject. Thus, it is considered that the probability of the ultrasound images of the two frames being similar to each other increases.

On the other hand, it is considered that the larger the change in the angle of the ultrasound probe 21C in the period from generation of one of the ultrasound images of the two frames different from each other to the end of generation of the other one, the farther the scan cross-sections represented by the ultrasound images of the two frames are from each other in a deep portion of the subject. Thus, it is considered that the probability of the ultrasound images of the two frames being similar to each other decreases.

Based on such an idea, the similarity estimation unit 31C sets an ultrasound image of a reference frame and an ultrasound image of an estimation-target frame from the ultrasound images of the first frame group. The similarity estimation unit 31C calculates a change between the angle A of the ultrasound probe 21C corresponding to the set ultrasound image of the reference frame and the angle A of the ultrasound probe 21C corresponding to the ultrasound image of the estimation-target frame by using the change-in-angle calculation unit 36. Based on the calculated change in angle, the similarity estimation unit 31C estimates whether or not the ultrasound image of the estimation-target frame is similar to the ultrasound image of the reference frame.

The similarity estimation unit 31C has, for example, a predetermined change-in-angle threshold, such as 5 degrees, for the change in the angle of the ultrasound probe 21C calculated by the change-in-angle calculation unit 36. If the change in the angle calculated by the change-in-angle calculation unit 36 is smaller than the predetermined change-in-angle threshold, the similarity estimation unit 31C estimates that the ultrasound image of the estimation-target frame is similar to the ultrasound image of the reference frame. If the change in the angle calculated by the change-in-angle calculation unit 36 is larger than or equal to the predetermined change-in-angle threshold, the similarity estimation unit 31C estimates that the ultrasound image of the estimation-target frame is dissimilar from the ultrasound image of the reference frame.

Similarly to the second frame group formation unit 32 in the second and third embodiments, the second frame group formation unit 32 removes the ultrasound image of the estimation-target frame estimated to be similar to the ultrasound image of the reference frame by the similarity estimation unit 31C from the ultrasound images of the first frame group, and forms the second frame group of ultrasound images using the ultrasound images of the rest of the frames.

As described above, the ultrasound diagnostic apparatus according to the fourth embodiment calculates a change in the angle of the ultrasound probe 21C between ultrasound images of two frames different from each other among the ultrasound images of the first frame group, and removes, based on the calculated change in the angle, the ultrasound image from the ultrasound images of the first frame group. In this manner, the ultrasound diagnostic apparatus according to the fourth embodiment can form the second frame group of ultrasound images constituted by ultrasound images of a smaller number of frames than the number of frames of the ultrasound images of the first frame group by thinning out ultrasound images of some frames from the ultrasound images of the first embodiment as in the second and third embodiments. Thus, when selecting the ultrasound image UD of the measurement frame, the user need not check ultrasound images of many frames. Consequently, the load imposed on the user can be reduced.

Since the second frame group of ultrasound images is constituted by ultrasound images of frames dissimilar from each other, the user can easily select the ultrasound image UD of the suitable measurement frame from the ultrasound image UC of at least one candidate frame. Consequently, the accuracy of urine volume measurement can be increased.

When the image memory 7 stores ultrasound images of a series of frames input from the outside, as the ultrasound images of the first frame group, the angle A of the ultrasound probe corresponding to each of the ultrasound images of the first frame group is also stored in the image memory 7 together. In this manner, the configuration of the fourth embodiment is applicable. In this case, the accuracy of urine volume measurement can be increased while reducing the load imposed on the user.

The second frame group formation unit 32 removes the ultrasound image of the estimation-target frame estimated to be similar to the ultrasound image of the reference frame by the similarity estimation unit 31C from the ultrasound images of the first frame group. However, the second frame group formation unit 32 may have a predetermined angle threshold such as 45 degrees, for example, for the angle A of the ultrasound probe 21C and may remove the ultrasound image of the frame for which the angle A of the ultrasound probe 21C is greater than or equal to the predetermined angle threshold. When the ultrasound probe 21C is inclined at the angle A greater than the predetermined angle threshold, the urinary bladder region BR is often not correctly contained in the ultrasound image. Thus, by removing an ultrasound image of a frame for which the angle A of the ultrasound probe 21C is greater than or equal to the predetermined angle threshold from the ultrasound images of the first frame group, ultrasound images of frames more suitable for urine volume measurement can be included in the ultrasound images of the second frame group.

### Fifth Embodiment

Similarly to the frame thinning-out unit 8 in the first embodiment, the second frame group of ultrasound images is formed by extracting ultrasound images at predetermined intervals in time series from the ultrasound images of the first frame group. Then, similarly to the frame thinning-out unit 8A in the second embodiment, some ultrasound images are removed from the ultrasound images of the second frame group. In this manner, the number of frames of ultrasound images constituting the second frame group of ultrasound images can be reduced.

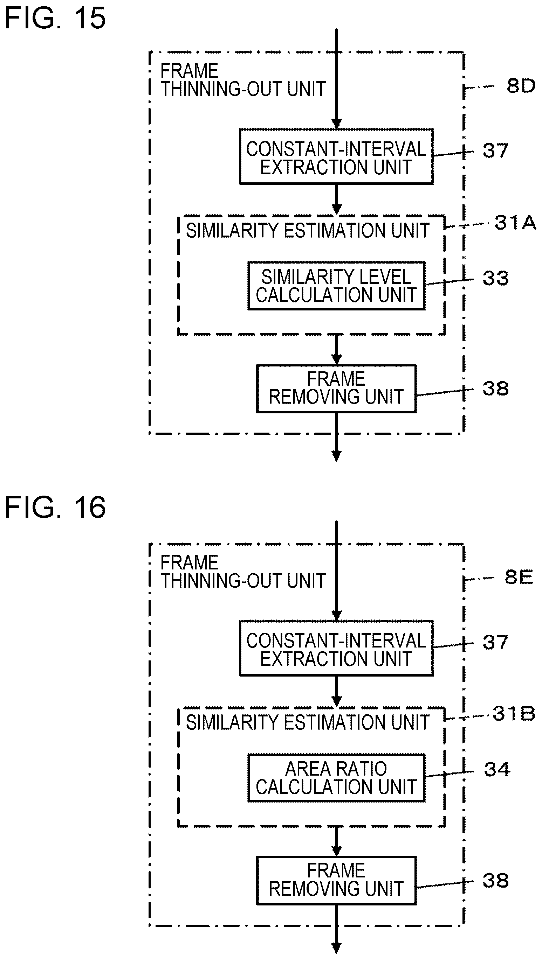

An ultrasound diagnostic apparatus according to the fifth embodiment is equivalent to the ultrasound diagnostic apparatus 1 according to the first embodiment illustrated in Fig. 1 that includes a frame thinning-out unit 8D illustrated in Fig. 15 instead of the frame thinning-out unit 8. The frame thinning-out unit 8D is configured such that a constant-interval extraction unit 37, the similarity estimation unit 31A, and a frame removing unit 38 are connected in series. The similarity estimation unit 31A includes the similarity level calculation unit 33.

The constant-interval extraction unit 37 is the same as the frame thinning-out unit 8 in the first embodiment. The constant-interval extraction unit 37 extracts an ultrasound image at predetermined intervals in time series from the ultrasound images of the first frame group stored in the image memory 7 to form the second frame group of ultrasound images.

The similarity estimation unit 31A is the same as the similarity estimation unit 31A in the second embodiment. The similarity estimation unit 31A sets an ultrasound image of a reference frame and an ultrasound image of an estimation-target frame from the ultrasound images of the second frame group formed by the constant-interval extraction unit 37. The similarity estimation unit 31A calculates a similarity level between the ultrasound image of the reference frame and the ultrasound image of the estimation-target frame by using the similarity level calculation unit 33. Based on the calculated similarity level, the similarity estimation unit 31A determines whether or not the ultrasound image of the estimation-target frame is similar to the ultrasound image of the reference frame.

The frame removing unit 38 removes the ultrasound image of the estimation-target frame estimated to be similar to the ultrasound image of the reference frame by the similarity estimation unit 31A from the ultrasound images of the second frame group.

As described above, in the ultrasound diagnostic apparatus according to the fifth embodiment, the frame removing unit 38 removes ultrasound images based on the similarity estimation performed by the similarity estimation unit 31A from the ultrasound images of the second frame group formed by the constant-interval extraction unit 37. Thus, the ultrasound diagnostic apparatus according to the fifth embodiment can further reduce the number of frames of the ultrasound images constituting the second frame group of ultrasound images and form the second frame group of ultrasound images using ultrasound images of frames dissimilar from each other. Thus, when the candidate frame extraction unit 11 extracts the ultrasound image UC of at least one candidate frame, the probability of extracting the ultrasound image of a more suitable frame increases. Thus, the accuracy of urine volume measurement can be increased while reducing the load imposed on the user in selecting the ultrasound image UD of the measurement frame.

Similarity estimation by the similarity estimation unit 31A and removal of the ultrasound image of the estimation-target frame similar to the ultrasound image of the reference frame by the frame removing unit 38 can be performed at a time on the ultrasound images of the second frame group formed by extracting ultrasound images separated at constant intervals in time series at a time from the ultrasound images of the first frame group by the constant-interval extraction unit 37. However, the ultrasound image extraction process by the constant-interval extraction unit 37, the similarity estimation process by the similarity estimation unit 31A, and the ultrasound image removal process by the frame removing unit 38 may be performed sequentially from the ultrasound image of the frame generated in the earliest timing among the ultrasound images of the first frame group.

### Sixth Embodiment

Instead of the similarity estimation unit 31A of the frame thinning-out unit 8D in the fifth embodiment, the similarity estimation unit 31B in the third embodiment may be included. As illustrated in Fig. 16, a frame thinning-out unit 8E in a sixth embodiment is configured such that the constant-interval extraction unit 37, the similarity estimation unit 31B, and the frame removing unit 38 are connected in series. The similarity estimation unit 31B includes the area ratio calculation unit 34.

In this case, the constant-interval extraction unit 37 extracts ultrasound images at predetermined intervals from the ultrasound images of the first frame group stored in the image memory 7 to form the second frame group of ultrasound images. The similarity estimation unit 31B calculates an area ratio of the urinary bladder region BR between the ultrasound image of the reference frame and the ultrasound image of the estimation-target frame. The frame removing unit 38 removes the ultrasound image of the estimation-target frame estimated to be similar to the ultrasound image of the reference frame based on the calculated area ratio, from the ultrasound images of the second frame group. Thus, similarly to the ultrasound diagnostic apparatus according to the fifth embodiment, the ultrasound diagnostic apparatus according to the sixth embodiment can increase the accuracy of urine volume measurement while reducing the load imposed on the user in selecting the ultrasound image UD of the measurement frame.

### Seventh Embodiment

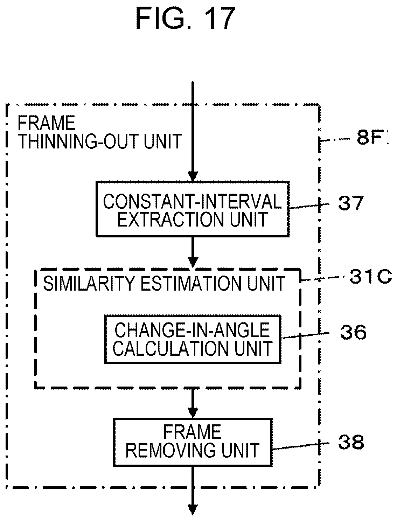

The ultrasound diagnostic apparatus 1C according to the fourth embodiment illustrated in Fig. 12 may include a frame thinning-out unit 8F illustrated in Fig. 17 instead of the frame thinning-out unit 8C. The frame thinning-out unit 8F is equivalent to the frame thinning-out unit 8D in the fifth embodiment illustrated in Fig. 15 that includes the similarity estimation unit 31C in the fourth embodiment illustrated in Fig. 14 instead of the similarity estimation unit 31A. The similarity estimation unit 31C includes the change-in-angle calculation unit 36.

In this case, the constant-interval extraction unit 37 extracts ultrasound images at predetermined intervals from the ultrasound images of the first frame group stored in the image memory 7 to form the second frame group of ultrasound images. The frame removing unit 38 removes, from the ultrasound images of the second frame group, the ultrasound image of the estimation-target frame estimated, by the similarity estimation unit 31C, to be similar to the ultrasound image of the reference frame based on the change in angle of the ultrasound probe 21 in a period from generation of the ultrasound image of the reference frame to the end of generation of the ultrasound image of the estimation-target frame. Thus, similarly to the ultrasound diagnostic apparatuses according to the fifth and sixth embodiments, the ultrasound diagnostic apparatuses according to the seventh embodiment can increase the accuracy of urine volume measurement while reducing the load imposed on the user in selecting the ultrasound image UD of the measurement frame.

### Eighth Embodiment

In the fifth embodiment, the constant-interval extraction unit 37 can perform the ultrasound image extraction process sequentially from the ultrasound image of the frame generated at the earliest timing among the ultrasound images of the first frame group. In this case, each time the similarity level is calculated by the similarity level calculation unit 33, the intervals at which the constant-interval extraction unit 37 extracts an ultrasound image from the ultrasound images of the first frame group in time series can be updated in accordance with the value of the calculated similarity level.

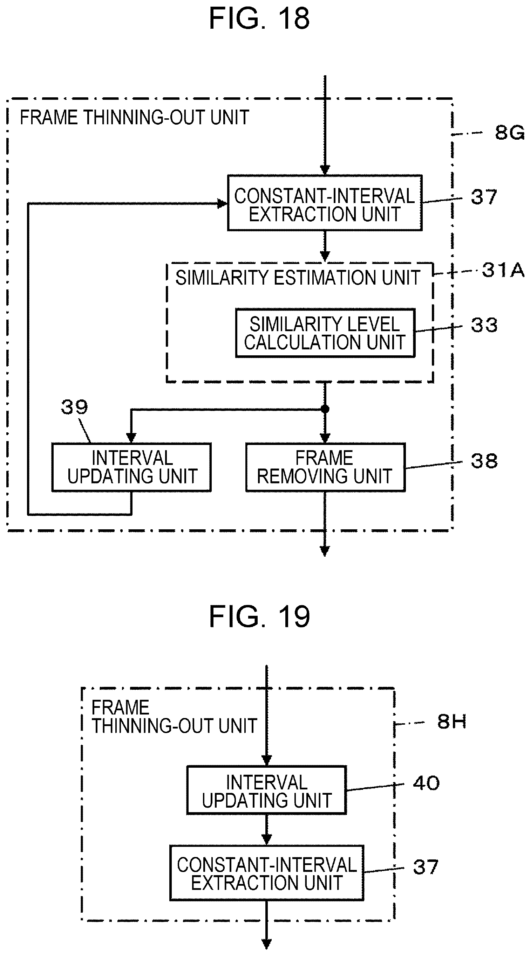

As illustrated in Fig. 18, a frame thinning-out unit 8G in the eighth embodiment is equivalent to the frame thinning-out unit 8D in the fifth embodiment illustrated in Fig. 15 to which an interval updating unit 39 is added. The interval updating unit 39 is connected to the similarity estimation unit 31A and the constant-interval extraction unit 37.

The interval updating unit 39 has a predetermined similarity level reference value for the similarity level calculated by the similarity level calculation unit 33. The interval updating unit 39 predicts that the greater the similarity level is than the predetermined similarity level reference value, the higher the similarity level between ultrasound images of a series of frames that follow, in time series, the ultrasound image of the estimation-target frame for which the similarity level is calculated, and updates the intervals at which the constant-interval extraction unit 37 extracts the ultrasound image in time series to a greater value. The interval updating unit 39 predicts that the smaller the similarity level calculated by the similarity level calculation unit 33 is than the predetermined similarity level reference value, the lower the similarity level between ultrasound images of a series of frames that follow, in time series, the ultrasound image of the estimation-target frame for which the similarity level is calculated, and updates the intervals at which the constant-interval extraction unit 37 extracts the ultrasound image in time series to a smaller value.

In this manner, by efficiently extracting the ultrasound images from the ultrasound images of the first frame group by taking into account the similarity level calculated by the similarity level calculation unit 33, the second frame group of ultrasound images can be formed, and the ultrasound images of the frames suitable for urine volume measurement can be set as the ultrasound images of the second frame group. Thus, the second frame group of ultrasound images can be formed more quickly, and the accuracy of urine volume measurement can be increased.

Description is given such that the configuration of the eighth embodiment is applied to the ultrasound diagnostic apparatus according to the fifth embodiment. However, the configuration of the eighth embodiment is similarly applicable to the ultrasound diagnostic apparatus according to the sixth embodiment and the ultrasound diagnostic apparatus according to the seventh embodiment.

When the configuration of the eighth embodiment is applied to the ultrasound diagnostic apparatus according to the sixth embodiment, the interval updating unit 39 can update the intervals at which the constant-interval extraction unit 37 extracts the ultrasound image in time series, based on the area ratio of the urinary bladder region BR between the ultrasound image of the reference frame and the ultrasound image of the estimation-target frame among the ultrasound images of the first frame group.

In this case, the interval updating unit 39 predicts that as the area ratio between the urinary bladder region BR in the ultrasound image of the reference frame and the urinary bladder region BR in the ultrasound image of the estimation-target frame is closer to 1, ultrasound images of a series of frames that follow, in time series, the ultrasound image of the estimation-target frame used in the calculation of the area ratio are more similar to each other, and updates the intervals at which the constant-interval extraction unit 37 extracts the ultrasound image in time series to a greater value. The interval updating unit 39 predicts that as the calculated area ratio is smaller or greater than 1, ultrasound images of a series of frames that follow, in time series, the ultrasound image of the estimation-target frame used in the calculation of the area ratio are dissimilar from each other, and updates the intervals at which the constant-interval extraction unit 37 extracts the ultrasound image in time series to a smaller value.

When the configuration of the eighth embodiment is applied to the ultrasound diagnostic apparatus according to the seventh embodiment, the interval updating unit 39 can update the intervals at which the constant-interval extraction unit 37 extracts the ultrasound image in time series, based on the change between the angle A of the ultrasound probe in the ultrasound image of the reference frame and the angle A of the ultrasound probe for the ultrasound image of the estimation-target frame among the ultrasound images of the first frame group.

In this case, the interval updating unit 39 has a change-in-angle reference value for the change in the angle of the ultrasound probe between the ultrasound image of the reference frame and the ultrasound image of the estimation-target frame. The interval updating unit 39 determines that the smaller the calculated change in the angle is than the change-in-angle reference value, the higher the inclination speed of the ultrasound probe is, and updates the intervals at which the constant-interval extraction unit 37 extracts the ultrasound image in time series to a greater value. The interval updating unit 39 determines that the greater the calculated change in the angle is than the change-in-angle reference value, the lower the inclination speed of the ultrasound probe is, and updates the intervals at which the constant-interval extraction unit 37 extracts the ultrasound image in time series to a smaller value.

### Ninth Embodiment

In the first embodiment, the intervals at which the frame thinning-out unit 8 extracts the ultrasound image from the ultrasound images of the first frame group in time series can be updated in accordance with the time taken for one scan of the urinary bladder B.

An ultrasound diagnostic apparatus according to a ninth embodiment is equivalent to the ultrasound diagnostic apparatus 1 according to the first embodiment illustrated in Fig. 1 that includes a frame thinning-out unit 8H illustrated in Fig. 19 instead of the frame thinning-out unit 8. The frame thinning-out unit 8H extracts an ultrasound image at predetermined intervals from the ultrasound images of the first frame group stored in the image memory 7 to form the second frame group of ultrasound images. As illustrated in Fig. 19, the frame thinning-out unit 8H includes an interval updating unit 40 and the constant-interval extraction unit 37 in the fifth embodiment illustrated in Fig. 15. The constant-interval extraction unit 37 is connected to the interval updating unit 40.

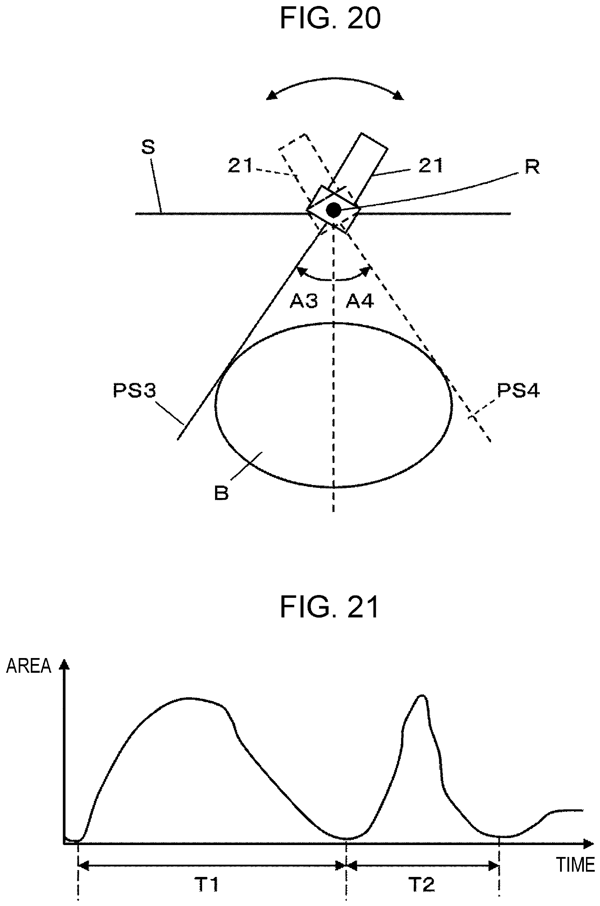

When the user operates the ultrasound probe 21 to scan the urinary bladder B, the user can scan the urinary bladder B using, for example, a swing method in which the ultrasound probe 21 is inclined on the body surface S of the subject while the position of the ultrasound probe 21 is fixed. When the user scans the urinary bladder B using the swing method, the user inclines the ultrasound probe 21 back and forth on the body surface S of the subject between the angle A of the ultrasound probe 21 at which a scan cross-section PS3 that passes through one end of the urinary bladder B is imaged and the angle A of the ultrasound probe 21 at which a scan cross-section PS4 that passes through the other end of the urinary bladder B with respect to the rotational axis R that is parallel to the arrangement direction of the transducer array 2, for example, as illustrated in Fig. 20.

When the urinary bladder B is scanned using the swing method illustrated in Fig. 20, the area of the urinary bladder region BR calculated in the ultrasound images of the generated frames takes the local minimum value at an angle A3 at which the scan cross-section PS3 is imaged and at an angle A4 at which the scan cross-section PS4 is imaged, and takes the local maximum value at the angle A at which a scan cross-section that passes through the center of the urinary bladder B is imaged. Thus, the calculated area of the urinary bladder region BR changes as time passes as illustrated in Fig. 21, for example. Fig. 21 indicates that in each of a time T1 and a time T2 shorter than the time T1, the area of the urinary bladder region BR changes in an order of a local minimum value, a local maximum value, and a local minimum value, and that the ultrasound probe 21 is inclined so that the angle A of the ultrasound probe 21 changes from the angle A3 at which the scan cross-section PS3 is imaged to the angle A4 at which the scan cross-section PS4 is imaged or from the angle A4 at which the scan cross-section PS4 is imaged to the angle A3 at which the scan cross-section PS3 is imaged.

Thus, from a change in the calculated area of the urinary bladder region BR in time series, the time T1 taken for the angle A of the ultrasound probe 21 to change from the angle A3 to the angle A4 and the time T2 taken for the angle A of the ultrasound probe 21 to change from the angle A4 to the angle A3 as a result of inclining the ultrasound probe 21 can be calculated.

The interval updating unit 40 performs image analysis on each of the ultrasound images of the first frame group to calculate the area of the urinary bladder region BR. The interval updating unit 40 analyzes a time-series change in the calculated area of the urinary bladder region BR to calculate the time T1 or the time T2 taken for the angle A of the ultrasound probe 21 to change from the angle A3 to the angle A4 or to change from the angle A4 to the angle A3 as the time taken for one scan of the urinary bladder B. Based on the calculated time T1 or T2, the interval updating unit 40 updates the intervals at which the constant-interval extraction unit 37 extracts an ultrasound image in time series from the ultrasound images of the first frame group.

For example, the interval updating unit 40 updates the intervals at which the frame thinning-out unit 8 extracts an ultrasound image in time series to a value obtained by dividing the time T1 by a constant value for ultrasound images of frames generated in the time T1 among the ultrasound images of the first frame group, and updates the intervals at which the constant-interval extraction unit 27 37 extracts an ultrasound image from the ultrasound images of the first frame group in time series to a value obtained by dividing the time T2 by the same constant value for ultrasound images of frames generated in the time T2.

The constant-interval extraction unit 37 extracts an ultrasound image at the intervals updated by the interval updating unit 40 in time series from the ultrasound images of the first frame group to form the second frame group of ultrasound images.

As described above, as the time T1 taken for the angle A of the ultrasound probe 21 to change from the angle A3 to the angle A4 or the time T2 taken for the angle A of the ultrasound probe 21 to change from the angle A4 to the angle A3 becomes longer, that is, as the moving speed of the ultrasound probe 21 becomes slower, longer intervals at which the frame thinning-out unit 8 extracts an ultrasound image in time series can be set by the ultrasound diagnostic apparatus according to the ninth embodiment, and as the time T1 or the time T2 becomes shorter, that is, the moving speed of the ultrasound probe 21 becomes faster, shorter intervals at which the frame thinning-out unit 8 extracts an ultrasound image in time series can be set by the ultrasound diagnostic apparatus according to the ninth embodiment.

Thus, the second frame group of ultrasound images can be formed by extracting more ultrasound images of frames similar to each other from the ultrasound images of the first frame group. This makes it easier for the user to select an ultrasound image of a suitable frame when selecting the ultrasound image UD of the measurement frame from the ultrasound image UC of at least one candidate frame extracted from the ultrasound images of the second frame group. Thus, the ultrasound diagnostic apparatus according to the ninth embodiment can further increase the accuracy of urine volume measurement while further reducing the load imposed on the user.

An example in which the user scans the urinary bladder B using the swing method has been described. Alternatively, a slide method in which the ultrasound probe 21 is translated on the body surface S of the subject while keeping the angle A of the ultrasound probe 21 constant can also be used for scanning the urinary bladder B.

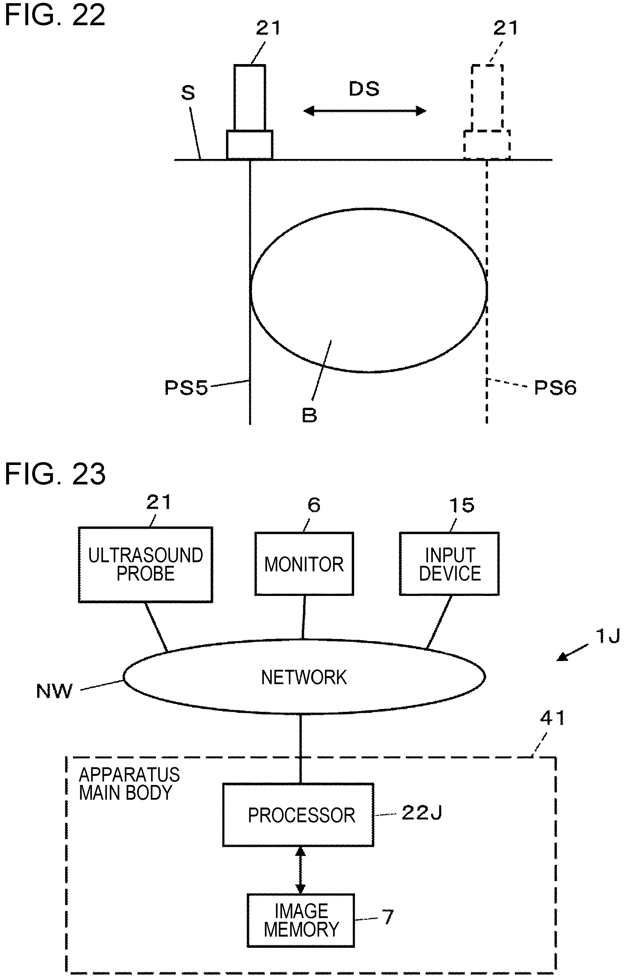

For example, as illustrated in Fig. 22, when the user scans the urinary bladder B using the slide method, the user moves the ultrasound probe 21 back and forth between a position at which a scan cross-section PS5 that passes through one end of the urinary bladder B in a sliding direction DS is imaged and a position at which a scan cross-section PS6 that passes through the other end of the urinary bladder B in the sliding direction DS is imaged, where the sliding direction DS is a direction in which the ultrasound probe 21 is translated on the body surface S of the subject.

In this case, the area of the urinary bladder region BR in the ultrasound images of the generated frames takes the local minimum values at the position of the ultrasound probe 21 where the scan cross-sections PS5 is imaged and at the position of the ultrasound probe 21 where the scan cross-sections PS6 is imaged, and takes the local maximum value at the position of the ultrasound probe 21 where a scan cross-section that passes through the center of the urinary bladder region B is imaged. Thus, as in the case where the urinary bladder B is scanned using the swing method, the area of the urinary bladder region BR changes as illustrated in Fig. 21. Thus, even when the urinary bladder B is scanned using the slide method, the interval updating unit 40 can update the intervals at which the constant-interval extraction unit 37 extracts an ultrasound image in time series in accordance with the time taken for translation of the ultrasound probe 21 in the sliding direction DS between the position at which the scan cross-sections PS5 is imaged and the position at which the scan cross-sections PS6 is imaged.

Description is given that the area of the urinary bladder region BR changes as time passes as illustrated in Fig. 21. For example, the largest diameter of the urinary bladder region BR in the depth direction of the ultrasound image and the largest diameter of the urinary bladder region BR in the horizontal direction of the ultrasound image also change as time passes as illustrated in Fig. 21. Thus, for example, the interval updating unit 40 may calculate the largest diameter of the urinary bladder region BR in the depth direction or the horizontal direction, and update the intervals at which the constant-interval extraction unit 37 extracts ultrasound images in time series by using the time-series change in the largest diameter of the urinary bladder region BR in the depth direction or the horizontal direction instead of the time-series change in the area of the urinary bladder region BR.

The interval updating unit 40 may calculate a maximum distance among distances between any two points on the outline of the urinary bladder region BR as the largest diameter of the urinary bladder region BR without limiting the direction in which the diameter of the urinary bladder region BR is measured. Since the largest diameter of the urinary bladder region BR calculated in this manner also changes as time passes as illustrated in Fig. 21, the interval updating unit 40 can also update the intervals at which the constant-interval extraction unit 37 extracts an ultrasound image in time series using this time-series change in the largest diameter.

When the urinary bladder B is scanned using the swing method, the angle A of the ultrasound probe 21 changes periodically in time series, for example, changes from 0 to the angle A3, changes from the angle A3 to 0, changes from 0 to the angle A4, changes from the angle A4 to 0, and changes again from 0 to the angle A3. Thus, for example, the angle sensor 35 in the fourth embodiment illustrated in Fig. 12 may be attached to the ultrasound probe 21. The interval updating unit 40 analyzes the time-series change in the angle A of the ultrasound probe 21 detected by the angle sensor 35 to acquire the time taken for the angle A of the ultrasound probe 21 to change. Based on the acquired time, the interval updating unit 40 may update the intervals at which the constant-interval extraction unit 37 extracts the ultrasound image in time series.

The configuration of the ninth embodiment can be applied not only to the ultrasound diagnostic apparatus 1 according to the first embodiment but also to the ultrasound diagnostic apparatuses according to the second to eighth embodiments in the same manner.

### Tenth Embodiment

The ultrasound diagnostic apparatus 1 according to the first embodiment has a configuration in which the monitor 6, the input device 15, and the ultrasound probe 21 are directly connected to the processor 22. However, for example, the monitor 6, the input device 15, the ultrasound probe 21, and the processor 22 may be connected indirectly to each other via a network.

As illustrated in Fig. 23, in an ultrasound diagnostic apparatus 1J according to a tenth embodiment, the monitor 6, the input device 15, and the ultrasound probe 21 are connected to an ultrasound diagnostic apparatus main body 41 via a network NW. The ultrasound diagnostic apparatus main body 41 is equivalent to the ultrasound diagnostic apparatus 1 according to the first embodiment illustrated in Fig. 1 from which the monitor 6, the input device 15, and the ultrasound probe 21 are omitted, and is constituted by the image memory 7 and a processor 22J.

Even if the ultrasound diagnostic apparatus 1J has such a configuration, similarly to the ultrasound diagnostic apparatus 1 according to the first embodiment, the second frame group of ultrasound images is formed by thinning out ultrasound images of some of frames from the ultrasound images of the first frame group stored in the image memory 7, the ultrasound image UC of at least one candidate frame is extracted from the ultrasound images of the second frame group, and the ultrasound image UD of the measurement frame is selected from the ultrasound image UC of the at least one candidate frame. The urine volume measurement in the urinary bladder B is performed based on the ultrasound image of the selected measurement frame. Thus, the accuracy of urine volume measurement can be increased while reducing the load imposed on the user in selecting the ultrasound image UD of the measurement frame.

Since the monitor 6, the input device 15, and the ultrasound probe 21 are connected to the ultrasound diagnostic apparatus main body 41 via the network NW, the ultrasound diagnostic apparatus main body 41 can be used as a so-called remote server. Thus, for example, the user can examine the subject by preparing the monitor 6, the input device 15, and the ultrasound probe 21, so that convenience at the time of ultrasonic diagnosis can be improved.

For example, when a thin portable computer called a so-called tablet is used as the monitor 6 and the input device 15, the user can more easily perform urine volume measurement. Thus, convenience of the urine volume measurement can be further improved.

The monitor 6, the input device 15, and the ultrasound probe 21 are connected to the ultrasound diagnostic apparatus main body 41 via the network NW. At this time, the monitor 6, the input device 15, and the ultrasound probe 21 may be connected to the network NW with cables or wirelessly.

Description is given such that the configuration of the tenth embodiment is applied to the first embodiment. However, the configuration of the tenth embodiment is similarly applicable to the second to ninth embodiments.

### Reference Signs List

- 1, 1C, 1J: ultrasound diagnostic apparatus
- 2: transducer array
- 3: transmission/reception circuit
- 4: image generation unit
- 5: display control unit
- 6: monitor
- 7: image memory
- 8, 8A, 8B, 8C, 8D, 8E, 8F, 8G, 8H: frame thinning-out unit
- 9: urinary bladder extraction unit
- 10: feature quantity calculation unit
- 11: candidate frame extraction unit
- 12: measurement frame selection unit
- 13: urine volume measurement unit
- 14, 14C: device control unit
- 15: input device
- 21, 21C: ultrasound probe
- 22, 22C, 22J: processor
- 23: pulser
- 24: amplifier
- 25: AD converter
- 26: beamformer
- 27: signal processing unit
- 28: DSC
- 29: image processing unit
- 31A, 31B, 31C: similarity estimation unit
- 32: second frame group formation unit
- 33: similarity level calculation unit
- 34: area ratio calculation unit
- 35: angle sensor
- 36: change-in-angle calculation unit
- 37: constant-interval extraction unit
- 38: frame removing unit
- 39, 40: interval updating unit
- 41: ultrasound diagnostic apparatus main body
- A, A3, A4: angle
- B: urinary bladder
- BR: urinary bladder region
- DS: sliding direction
- E: ellipsoid
- J: urine volume
- LX, LY, LZ: largest diameter
- PS1, PS2, PS3, PS4, PS5, PS6: scan cross-section
- R: rotational axis
- S: body surface
- T1, T2: time
- U1, U2, U3, UC, UD: ultrasound image

## Claims

1. An ultrasound diagnostic apparatus comprising:
an image memory (7) configured to store a first frame group of ultrasound images;
a frame thinning-out unit (8) configured to thin out ultrasound images of some frames from the ultrasound images of the first frame group to form a second frame group of ultrasound images, the second frame group being constituted by a smaller number of frames of ultrasound images;
a urinary bladder extraction unit (9) configured to extract a urinary bladder region from each of the ultrasound images of the second frame group;
a feature quantity calculation unit (10) configured to calculate a feature quantity related to the urinary bladder region extracted for each of the ultrasound images of the second frame group by the urinary bladder extraction unit;
a candidate frame extraction unit (11) configured to extract, based on the feature quantity calculated by the feature quantity calculation unit, an ultrasound image of at least one candidate frame that serves as a candidate subjected to measurement from the ultrasound images of the second frame group;
a monitor (6) configured to display the ultrasound image of the at least one candidate frame extracted by the candidate frame extraction unit;
an input device (15) with which a user performs an input operation;
a measurement frame selection unit (12) configured to select, in accordance with the input operation performed by the user via the input device, an ultrasound image of a measurement frame that serves as a target subjected to measurement, from the ultrasound image of the at least one candidate frame displayed on the monitor;
a urine volume measurement unit (13) configured to analyze the ultrasound image of the measurement frame selected by the measurement frame selection unit to measure a urine volume; and
wherein the frame thinning-out unit is configured to extract an ultrasound image at predetermined intervals in time series from the ultrasound images of the first frame group and remove ultrasound images of rest of the frames from the ultrasound images of the first frame group to form the second frame group of ultrasound images, and
wherein the frame thinning-out unit includes
a similarity estimation unit (31A, 31B, 31C) configured to estimate whether or not ultrasound images of two frames are similar to each other, and
a frame removing unit (38) configured to remove, from the ultrasound images of the second frame group, an ultrasound image of a frame that is consecutive in time series to the extracted ultrasound image of one frame and that is estimated to be similar to the extracted ultrasound image of the one frame by the similarity estimation unit.

2. The ultrasound diagnostic apparatus according to claim 1, wherein the predetermined intervals are intervals of a predetermined number of frames or predetermined time intervals.

3. The ultrasound diagnostic apparatus according to claim 1, wherein
the similarity estimation unit
includes a similarity level calculation unit (33) configured to analyze the ultrasound images of the two frames to calculate a similarity level between the ultrasound images of the two frames, and
is configured to estimate that the ultrasound images of the two frames are similar to each other in a case where the similarity level calculated by the similarity level calculation unit is greater than or equal to a predetermined similarity level threshold, and estimate that the ultrasound images of the two frames are dissimilar from each other in a case where the similarity level calculated by the similarity level calculation unit is less than the predetermined similarity level threshold.

4. The ultrasound diagnostic apparatus according to claim 1, wherein
the similarity estimation unit
includes an area ratio calculation unit (34) configured to analyze the ultrasound images of the two frames to calculate an area ratio between urinary bladder regions in the respective ultrasound images of the two frames, and
is configured to estimate that the ultrasound images of the two frames are similar to each other in a case where the area ratio calculated by the area ratio calculation unit is within a predetermined area ratio range, and estimate that the ultrasound images of the two frames are dissimilar from each other in a case where the area ratio calculated by the area ratio calculation unit is out of the predetermined area ratio range.

5. The ultrasound diagnostic apparatus according to claim 1, further comprising:
an ultrasound probe (21) configured to transmit an ultrasonic beam to a subject and receive an ultrasonic beam from the subject; and
an angle sensor (35) attached to the ultrasound probe and configured to detect an angle of the ultrasound probe, wherein
the similarity estimation unit
includes a change-in-angle calculation unit (36) configured to calculate, based on the angle of the ultrasound probe detected by the angle sensor, a change in the angle of the ultrasound probe during capturing of the ultrasound images of the two frames, and
is configured to estimate that the ultrasound images of the two frames are similar to each other in a case where the change in the angle calculated by the change-in-angle calculation unit is smaller than a predetermined change-in-angle threshold, and estimate that the ultrasound images of the two frames are dissimilar from each other in a case where the change in the angle calculated by the change-in-angle calculation unit is greater than or equal to the predetermined change-in-angle threshold.

6. The ultrasound diagnostic apparatus according to any one of claims 3 to 5, wherein the frame thinning-out unit updates the predetermined intervals, based on the similarity level calculated by the similarity level calculation unit, the area ratio calculated by the area ratio calculation unit, or the change in the angle calculated by the change-in-angle calculation unit.

7. The ultrasound diagnostic apparatus according to any one of claims 1 to 6, wherein the frame thinning-out unit updates the predetermined intervals, based on a processing speed of the ultrasound diagnostic apparatus.

8. The ultrasound diagnostic apparatus according to any one of claims 1 to 7, wherein the frame thinning-out unit updates the predetermined intervals, based on a time taken for scanning of a urinary bladder.

9. The ultrasound diagnostic apparatus according to claim 1, wherein
the frame thinning-out unit
includes a similarity estimation unit configured to estimate whether or not ultrasound images of two frames are similar to each other, and
is configured to form the second frame group of ultrasound images using ultrasound images of frames that are left after removal of an ultrasound image of a frame that is consecutive to an ultrasound image of each frame of the ultrasound images of the first frame group and is estimated to be similar to the ultrasound image of the frame by the similarity estimation unit.

10. The ultrasound diagnostic apparatus according to claim 9, wherein
the similarity estimation unit
includes a similarity level calculation unit (33) configured to analyze the ultrasound images of the two frames to calculate a similarity level between the ultrasound images of the two frames, and
is configured to estimate that the ultrasound images of the two frames are similar to each other in a case where the similarity level calculated by the similarity level calculation unit is greater than or equal to a predetermined similarity level threshold, and estimate that the ultrasound images of the two frames are dissimilar from each other in a case where the similarity level calculated by the similarity level calculation unit is less than the predetermined similarity level threshold; or
wherein
the similarity estimation unit
includes an area ratio calculation unit (34) configured to analyze the ultrasound images of the two frames to calculate an area ratio between urinary bladder regions in the respective ultrasound images of the two frames, and
is configured to estimate that the ultrasound images of the two frames are similar to each other in a case where the area ratio calculated by the area ratio calculation unit is within a predetermined area ratio range, and estimate that the ultrasound images of the two frames are dissimilar from each other in a case where the area ratio calculated by the area ratio calculation unit is out of the predetermined area ratio range.

11. The ultrasound diagnostic apparatus according to claim 9, further comprising:
an ultrasound probe (21) configured to transmit an ultrasonic beam to a subject and receive an ultrasonic beam from the subject; and
an angle sensor (35) attached to the ultrasound probe and configured to detect an angle of the ultrasound probe, wherein
the similarity estimation unit
includes a change-in-angle calculation unit configured to calculate, based on the angle of the ultrasound probe detected by the angle sensor, a change in the angle of the ultrasound probe during capturing of the ultrasound images of the two frames, and
is configured to estimate that the ultrasound images of the two frames are similar to each other in a case where the change in the angle calculated by the change-in-angle calculation unit is smaller than a predetermined change-in-angle threshold, and estimate that the ultrasound images of the two frames are dissimilar from each other in a case where the change in the angle calculated by the change-in-angle calculation unit is greater than or equal to the predetermined angle-of-change threshold.

12. A method for controlling an ultrasound diagnostic apparatus, comprising:
thinning out ultrasound images of some frames from ultrasound images of a first frame group to form a second frame group of ultrasound images, the second frame group being constituted by a smaller number of frames than the first frame group of ultrasound images;
extracting a urinary bladder region from each of the ultrasound images of the second frame group;
calculating a feature quantity related to the urinary bladder region extracted for each of the ultrasound images of the second frame group;
extracting, based on the feature quantity, an ultrasound image of at least one candidate frame that serves as a candidate subjected to measurement from the ultrasound images of the second frame group;
displaying the ultrasound image of the at least one candidate frame on a monitor;
selecting, in accordance with an input operation performed by a user, an ultrasound image of a measurement frame that serves as a target subjected to measurement, from the ultrasound image of the at least one candidate frame displayed on the monitor;
analyzing the selected ultrasound image of the measurement frame to measure a urine volume; and
extracting, by a frame thinning-out unit (8), an ultrasound image at predetermined intervals in time series from the ultrasound images of the first frame group and remove ultrasound images of rest of the frames from the ultrasound images of the first frame group to form the second frame group of ultrasound images,
wherein the frame thinning-out unit includes a similarity estimation unit (31A, 31B, 31C) and a frame removing unit (38), and the method further comprising:
estimating, by the similarity estimation unit (31A, 31B, 31C), whether or not ultrasound images of two frames are similar to each other, and
removing, by the frame removing unit (38), from the ultrasound images of the second frame group, an ultrasound image of a frame that is consecutive in time series to the extracted ultrasound image of one frame and that is estimated to be similar to the extracted ultrasound image of the one frame by the similarity estimation unit.

13. A processor for an ultrasound diagnostic apparatus, the processor being configured to:
thin out ultrasound images of some frames from ultrasound images of a first frame group to form a second frame group of ultrasound images, the second frame group being constituted by a smaller number of frames than the first frame group of ultrasound images;
extract a urinary bladder region from each of the ultrasound images of the second frame group;
calculate a feature quantity related to the urinary bladder region extracted for each of the ultrasound images of the second frame group;
extract, based on the feature quantity, an ultrasound image of at least one candidate frame that serves as a candidate subjected to measurement from the ultrasound images of the second frame group;
display the ultrasound image of the at least one candidate frame on a monitor;
select, in accordance with an input operation performed by a user, an ultrasound image of a measurement frame that serves as a target subjected to measurement, from the ultrasound image of the at least one candidate frame displayed on the monitor;
analyze the ultrasound image of the measurement frame selected by the user to measure a urine volume; and
extract, by a frame thinning-out unit (8), an ultrasound image at predetermined intervals in time series from the ultrasound images of the first frame group and remove ultrasound images of rest of the frames from the ultrasound images of the first frame group to form the second frame group of ultrasound images,
wherein the frame thinning-out unit includes a similarity estimation unit (31A, 31B, 31C) and a frame removing unit (38), and the processor is further configured to:
estimate, by the similarity estimation unit (31A, 31B, 31C), whether or not ultrasound images of two frames are similar to each other, and
remove, by the frame removing unit (38), from the ultrasound images of the second frame group, an ultrasound image of a frame that is consecutive in time series to the extracted ultrasound image of one frame and that is estimated to be similar to the extracted ultrasound image of the one frame by the similarity estimation unit.

## Patentansprüche

1. Ultraschalldiagnosevorrichtung, umfassend:
einen Bildspeicher (7), der so konfiguriert ist, dass er eine erste Einzelbildgruppe von Ultraschallbildern speichert;
eine Einzelbild-Ausdünnungseinheit (8), die so konfiguriert ist, dass sie Ultraschallbilder einiger Einzelbilder aus den Ultraschallbildern der ersten Einzelbildgruppe ausdünnt, um eine zweite Einzelbildgruppe von Ultraschallbildern zu bilden, wobei die zweite Einzelbildgruppe durch eine kleinere Anzahl an Einzelbildern von Ultraschallbildern gebildet wird;
eine Harnblasen-Extraktionseinheit (9), die so konfiguriert ist, dass sie einen Harnblasenbereich aus jedem der Ultraschallbilder der zweiten Einzelbildgruppe extrahiert;
eine Merkmalsmengen-Berechnungseinheit (10), die so konfiguriert ist, dass sie eine Merkmalsmenge berechnet, die sich auf den Harnblasenbereich, der für jedes der Ultraschallbilder der zweiten Einzelbildgruppe von der Harnblasen-Extraktionseinheit extrahiert worden ist, bezieht;
eine Kandidateneinzelbild-Extraktionseinheit (11), die so konfiguriert ist, dass sie auf der Grundlage der von der Merkmalsmengen-Berechnungseinheit berechneten Merkmalsmenge ein Ultraschallbild von mindestens einem Kandidateneinzelbild, das als ein Kandidat, der Messung unterzogen wird, dient, aus den Ultraschallbildern der zweiten Einzelbildgruppe extrahiert;
einen Monitor (6), der so konfiguriert ist, dass er das Ultraschallbild des mindestens einen Kandidateneinzelbildes, das von der Kandidateneinzelbild-Extraktionseinheit extrahiert worden ist, anzeigt;
eine Eingabevorrichtung (15), mit der ein Benutzer eine Eingabebedienung durchführt;
eine Messeinzelbild-Auswahleinheit (12), die so konfiguriert ist, dass sie in Übereinstimmung mit der von dem Benutzer via die Eingabevorrichtung durchgeführten Eingabebedienung ein Ultraschallbild eines Messeinzelbildes, das als ein Ziel, das Messung unterzogen wird, dient, aus dem Ultraschallbild des mindestens einen Kandidateneinzelbildes, das auf dem Monitor angezeigt wird, auswählt; und
eine Urinvolumen-Messeinheit (13), die so konfiguriert ist, dass sie das Ultraschallbild des von der Messeinzelbild-Auswahleinheit ausgewählten Messeinzelbildes analysiert,
um ein Urinvolumen zu messen,
wobei die Einzelbild-Ausdünnungseinheit so konfiguriert ist, dass sie ein Ultraschallbild in vorbestimmten Abständen in Zeitreihe aus den Ultraschallbildern der ersten Einzelbildgruppe extrahiert und Ultraschallbilder von Rest der Einzelbilder aus den Ultraschallbildern der ersten Einzelbildgruppe entfernt, um die zweite Einzelbildgruppe von Ultraschallbildern zu bilden, und
wobei die Einzelbild-Ausdünnungseinheit enthält:
eine Ähnlichkeitsschätzungseinheit (31A, 31B, 31C), die so konfiguriert ist, dass sie schätzt, ob Ultraschallbilder von zwei Einzelbildern einander ähnlich sind oder nicht, und
eine Einzelbild-Entfernungseinheit (38), die so konfiguriert ist, dass sie ein Ultraschallbild eines Einzelbildes, das in Zeitreihe zu dem extrahierten Ultraschallbild eines Einzelbildes aufeinanderfolgend ist und das von der Ähnlichkeitsschätzungseinheit als ähnlich zu dem extrahierten Ultraschallbild des einen Einzelbildes geschätzt wird, aus den Ultraschallbildern der zweiten Einzelbildgruppe entfernt.

2. Ultraschalldiagnosevorrichtung nach Anspruch 1, wobei die vorbestimmten Abstände Abstände einer vorbestimmten Anzahl an Einzelbildern oder vorbestimmte Zeitabstände sind.

3. Ultraschalldiagnosevorrichtung nach Anspruch 1, wobei
die Ähnlichkeitsschätzungseinheit
eine Ähnlichkeitsniveau-Berechnungseinheit (33), die so konfiguriert ist, dass sie die Ultraschallbilder der zwei Einzelbilder analysiert, um ein Ähnlichkeitsniveau zwischen den Ultraschallbildern der zwei Einzelbilder zu berechnen, enthält, und
so konfiguriert ist, dass sie in einem Fall, in dem das von der Ähnlichkeitsniveau-Berechnungseinheit berechnete Ähnlichkeitsniveau größer oder gleich einem vorbestimmten Ähnlichkeitsniveau-Schwellenwert ist, schätzt, dass die Ultraschallbilder der zwei Einzelbilder einander ähnlich sind, und in einem Fall, in dem das von der Ähnlichkeitsniveau-Berechnungseinheit berechnete Ähnlichkeitsniveau kleiner als der vorbestimmte Ähnlichkeitsniveau-Schwellenwert ist, schätzt, dass die Ultraschallbilder der zwei Einzelbilder einander unähnlich sind.

4. Ultraschalldiagnosevorrichtung nach Anspruch 1, wobei
die Ähnlichkeitsschätzungseinheit
eine Flächenverhältnis-Berechnungseinheit (34), die so konfiguriert ist, dass sie die Ultraschallbilder der zwei Einzelbilder analysiert, um ein Flächenverhältnis zwischen Harnblasenbereichen in den jeweiligen Ultraschallbildern der zwei Einzelbilder zu berechnen, enthält, und
so konfiguriert ist, dass sie in einem Fall, in dem das von der Flächenverhältnis-Berechnungseinheit berechnete Flächenverhältnis innerhalb eines vorbestimmten Flächenverhältnisbereichs liegt, schätzt, dass die Ultraschallbilder der zwei Einzelbilder einander ähnlich sind, und in einem Fall, in dem das von der Flächenverhältnis-Berechnungseinheit berechnete Flächenverhältnis außerhalb des vorbestimmten Flächenverhältnisbereichs liegt, schätzt, dass die Ultraschallbilder der zwei Einzelbilder einander unähnlich sind.

5. Ultraschalldiagnosevorrichtung nach Anspruch 1, ferner umfassend:
eine Ultraschallsonde (21), die so konfiguriert ist, dass sie einen Ultraschallstrahl an eine Untersuchungsperson überträgt und einen Ultraschallstrahl von der Untersuchungsperson empfängt; und
einen Winkelsensor (35), der an der Ultraschallsonde angebracht ist und so konfiguriert ist, dass er einen Winkel der Ultraschallsonde detektiert, wobei die Ähnlichkeitsschätzungseinheit
eine Winkeländerungs-Berechnungseinheit (36), die so konfiguriert ist, dass sie auf der Grundlage des von dem Winkelsensor detektierten Winkels der Ultraschallsonde eine Änderung des Winkels der Ultraschallsonde während Aufnehmen der Ultraschallbilder der zwei Einzelbilder berechnet, enthält, und
so konfiguriert ist, dass sie in einem Fall, in dem die von der Winkeländerungs-Berechnungseinheit berechnete Änderung des Winkels kleiner als ein vorbestimmter Winkeländerungsschwellenwert ist, schätzt, dass die Ultraschallbilder der zwei Einzelbilder einander ähnlich sind, und in einem Fall, in dem die von der Winkeländerungs-Berechnungseinheit berechnete Änderung des Winkels größer oder gleich dem vorbestimmten Winkeländerungsschwellenwert ist, schätzt, dass die Ultraschallbilder der zwei Einzelbilder einander unähnlich sind.

6. Ultraschalldiagnosevorrichtung nach einem der Ansprüche 3 bis 5, wobei die Einzelbild-Ausdünnungseinheit die vorbestimmten Abstände auf der Grundlage des von der Ähnlichkeitsniveau-Berechnungseinheit berechneten Ähnlichkeitsniveaus, des von der Flächenverhältnis-Berechnungseinheit berechneten Flächenverhältnisses oder der von der Winkeländerungs-Berechnungseinheit berechneten Änderung des Winkels aktualisiert.

7. Ultraschalldiagnosevorrichtung nach einem der Ansprüche 1 bis 6, wobei die Einzelbild-Ausdünnungseinheit die vorbestimmten Abstände auf der Grundlage einer Verarbeitungsgeschwindigkeit der Ultraschalldiagnosevorrichtung aktualisiert.

8. Ultraschalldiagnosevorrichtung nach einem der Ansprüche 1 bis 7, wobei die Einzelbild-Ausdünnungseinheit die vorbestimmten Abstände auf der Grundlage einer Zeit, die zum Abtasten einer Harnblase benötigt wird, aktualisiert.

9. Ultraschalldiagnosevorrichtung nach Anspruch 1, wobei die Einzelbild-Ausdünnungseinheit
eine Ähnlichkeitsschätzungseinheit, die so konfiguriert ist, dass sie schätzt, ob Ultraschallbilder von zwei Einzelbildern einander ähnlich sind oder nicht, enthält, und so konfiguriert ist, dass sie die zweite Einzelbildgruppe von Ultraschallbildern unter Verwendung von Ultraschallbildern von Einzelbildern, die nach Entfernen eines Ultraschallbildes eines Einzelbildes, das zu einem Ultraschallbild jedes Einzelbildes der Ultraschallbilder der ersten Einzelbildgruppe aufeinanderfolgt und von der Ähnlichkeitsschätzungseinheit als ähnlich zu dem Ultraschallbild des Einzelbildes geschätzt wird, übrig bleiben, bildet.

10. Ultraschalldiagnosevorrichtung nach Anspruch 9, wobei
die Ähnlichkeitsschätzungseinheit
eine Ähnlichkeitsniveau-Berechnungseinheit (33), die so konfiguriert ist, dass sie die Ultraschallbilder der zwei Einzelbilder analysiert, um ein Ähnlichkeitsniveau zwischen den Ultraschallbildern der zwei Einzelbilder zu berechnen, enthält, und
so konfiguriert ist, dass sie in einem Fall, in dem das von der Ähnlichkeitsniveau-Berechnungseinheit berechnete Ähnlichkeitsniveau größer oder gleich einem vorbestimmten Ähnlichkeitsniveau-Schwellenwert ist, schätzt, dass die Ultraschallbilder der zwei Einzelbilder einander ähnlich sind, und in einem Fall, in dem das von der Ähnlichkeitsniveau-Berechnungseinheit berechnete Ähnlichkeitsniveau kleiner als der vorbestimmte Ähnlichkeitsniveau-Schwellenwert ist, schätzt, dass die Ultraschallbilder der zwei Einzelbilder einander unähnlich sind; oder
wobei
die Ähnlichkeitsschätzungseinheit
eine Flächenverhältnis-Berechnungseinheit (34), die so konfiguriert ist, dass sie die Ultraschallbilder der zwei Einzelbilder analysiert, um ein Flächenverhältnis zwischen Harnblasenbereichen in den jeweiligen Ultraschallbildern der zwei Einzelbilder zu berechnen, enthält, und
so konfiguriert ist, dass sie in einem Fall, in dem das von der Flächenverhältnis-Berechnungseinheit berechnete Flächenverhältnis innerhalb eines vorbestimmten Flächenverhältnisbereichs liegt, schätzt, dass die Ultraschallbilder der zwei Einzelbilder einander ähnlich sind, und in einem Fall, in dem das von der Flächenverhältnis-Berechnungseinheit berechnete Flächenverhältnis außerhalb des vorbestimmten Flächenverhältnisbereichs liegt, schätzt, dass die Ultraschallbilder der zwei Einzelbilder einander unähnlich sind.

11. Ultraschalldiagnosevorrichtung nach Anspruch 9, ferner umfassend:
eine Ultraschallsonde (21), die so konfiguriert ist, dass sie einen Ultraschallstrahl an eine Untersuchungsperson überträgt und einen Ultraschallstrahl von der Untersuchungsperson empfängt; und
einen Winkelsensor (35), der an der Ultraschallsonde angebracht ist und so konfiguriert ist, dass er einen Winkel der Ultraschallsonde detektiert, wobei die Ähnlichkeitsschätzungseinheit
eine Winkeländerungs-Berechnungseinheit, die so konfiguriert ist, dass sie auf der Grundlage des von dem Winkelsensor detektierten Winkels der Ultraschallsonde eine Änderung des Winkels der Ultraschallsonde während Aufnehmen der Ultraschallbilder der zwei Einzelbilder berechnet, enthält, und
so konfiguriert ist, dass sie in einem Fall, in dem die von der Winkeländerungs-Berechnungseinheit berechnete Änderung des Winkels kleiner als ein vorbestimmter Winkeländerungsschwellenwert ist, schätzt, dass die Ultraschallbilder der zwei Einzelbilder einander ähnlich sind, und in einem Fall, in dem die von der Winkeländerungs-Berechnungseinheit berechnete Änderung des Winkels größer oder gleich dem vorbestimmten Winkeländerungsschwellenwert ist, schätzt, dass die Ultraschallbilder der zwei Einzelbilder einander unähnlich sind.

12. Verfahren zum Steuern einer Ultraschalldiagnosevorrichtung, umfassend:
Ausdünnen von Ultraschallbildern einiger Einzelbilder aus Ultraschallbildern einer ersten Einzelbildgruppe, um eine zweite Einzelbildgruppe von Ultraschallbildern zu bilden, wobei die zweite Einzelbildgruppe aus einer kleineren Anzahl an Einzelbildern als die erste Einzelbildgruppe von Ultraschallbildern gebildet ist;
Extrahieren eines Harnblasenbereichs aus jedem der Ultraschallbilder der zweiten Einzelbildgruppe;
Berechnen einer Merkmalsmenge, die sich auf den extrahierten Harnblasenbereich für jedes der Ultraschallbilder der zweiten Einzelbildgruppe bezieht;
Extrahieren eines Ultraschallbildes von mindestens einem Kandidateneinzelbild, das als ein Kandidat, der Messung unterzogen wird, dient, aus den Ultraschallbildern der zweiten Einzelbildgruppe auf der Grundlage der Merkmalsmenge;
Anzeigen des Ultraschallbildes des mindestens einen Kandidateneinzelbildes auf einem Monitor;
Auswählen, in Übereinstimmung mit einer von einem Benutzer durchgeführten Eingabebedienung, eines Ultraschallbildes eines Messeinzelbildes, das als ein Ziel, das Messung unterzogen wird, dient, aus dem Ultraschallbild des mindestens einen Kandidateneinzelbildes, das auf dem Monitor angezeigt wird;
Analysieren des ausgewählten Ultraschallbildes des Messeinzelbildes, um ein Urinvolumen zu messen; und
Extrahieren, durch eine Einzelbild-Ausdünnungseinheit (8), eines Ultraschallbildes in vorbestimmten Abständen in Zeitreihe aus den Ultraschallbildern der ersten Einzelbildgruppe und Entfernen von Ultraschallbildern von Rest der Einzelbilder aus den Ultraschallbildern der ersten Einzelbildgruppe, um die zweite Einzelbildgruppe von Ultraschallbildern zu bilden,
wobei die Einzelbild-Ausdünnungseinheit eine Ähnlichkeitsschätzungseinheit (31A, 31B, 31C) und eine Einzelbild-Entfernungseinheit (38) enthält, und wobei das Verfahren ferner umfasst:
Schätzen, durch die Ähnlichkeitsschätzungseinheit (31A, 31B, 31C), ob Ultraschallbilder von zwei Einzelbildern einander ähnlich sind oder nicht, und
Entfernen, durch die Einzelbild-Entfernungseinheit (38), eines Ultraschallbildes eines Einzelbildes, das in Zeitreihe zu dem extrahierten Ultraschallbild eines Einzelbildes aufeinanderfolgend ist und das von der Ähnlichkeitsschätzungseinheit als ähnlich zu dem extrahierten Ultraschallbild des einen Einzelbildes geschätzt wird, aus den Ultraschallbildern der zweiten Einzelbildgruppe.

13. Prozessor für eine Ultraschalldiagnosevorrichtung, wobei der Prozessor so konfiguriert ist, dass er:
Ultraschallbilder einiger Einzelbilder aus Ultraschallbildern einer ersten Einzelbildgruppe ausdünnt, um eine zweite Einzelbildgruppe von Ultraschallbildern zu bilden, wobei die zweite Einzelbildgruppe aus einer kleineren Anzahl an Einzelbildern als die erste Einzelbildgruppe von Ultraschallbildern gebildet ist;
einen Harnblasenbereich aus jedem der Ultraschallbilder der zweiten Einzelbildgruppe extrahiert;
eine Merkmalsmenge berechnet, die sich auf den extrahierten Harnblasenbereich für jedes der Ultraschallbilder der zweiten Einzelbildgruppe bezieht;
auf der Grundlage der Merkmalsmenge ein Ultraschallbild von mindestens einem Kandidateneinzelbild, das als ein Kandidat, der Messung unterzogen wird, dient, aus den Ultraschallbildern der zweiten Einzelbildgruppe extrahiert;
das Ultraschallbild des mindestens einen Kandidateneinzelbildes auf einem Monitor anzeigt;
in Übereinstimmung mit einer von einem Benutzer durchgeführten Eingabebedienung ein Ultraschallbild eines Messeinzelbildes, das als ein Ziel, das Messung unterzogen wird, dient, aus dem Ultraschallbild des mindestens einen Kandidateneinzelbildes, das auf dem Monitor angezeigt wird, auswählt;
das Ultraschallbild des von dem Benutzer ausgewählten Messeinzelbildes analysiert, um ein Urinvolumen zu messen; und
durch eine Einzelbild-Ausdünnungseinheit (8) ein Ultraschallbild in vorbestimmten Abständen in Zeitreihe aus den Ultraschallbildern der ersten Einzelbildgruppe extrahiert und Ultraschallbilder von Rest der Einzelbilder aus den Ultraschallbildern der ersten Einzelbildgruppe entfernt, um die zweite Einzelbildgruppe von Ultraschallbildern zu bilden,
wobei die Einzelbild-Ausdünnungseinheit eine Ähnlichkeitsschätzungseinheit (31A, 31B, 31C) und eine Einzelbild-Entfernungseinheit (38) enthält, und der Prozessor ferner so konfiguriert ist, dass er:
durch die Ähnlichkeitsschätzungseinheit (31A, 31B, 31C) schätzt, ob Ultraschallbilder von zwei Einzelbildern einander ähnlich sind oder nicht, und
durch die Einzelbild-Entfernungseinheit (38) ein Ultraschallbild eines Einzelbildes, das in Zeitreihe zu dem extrahierten Ultraschallbild eines Einzelbildes aufeinanderfolgend ist und das durch die Ähnlichkeitsschätzungseinheit als ähnlich zu dem extrahierten Ultraschallbild des einen Einzelbildes geschätzt wird, aus den Ultraschallbildern der zweiten Einzelbildgruppe entfernt.

## Revendications

1. Appareil de diagnostic par ultrasons comprenant :
une mémoire d'images (7) configurée pour stocker un premier groupe de trames d'images ultrasonores ;
une unité de réduction du nombre de trames (8) configurée pour réduire le nombre des images ultrasonores de certaines trames parmi les images ultrasonores du premier groupe de trames pour former un deuxième groupe de trames d'images ultrasonores, le deuxième groupe de trames étant constitué d'un nombre réduit de trames d'images ultrasonores ;
une unité d'extraction de vessie urinaire (9) configurée pour extraire une région de vessie urinaire à partir de chacune des images ultrasonores du deuxième groupe de trames ;
une unité de calcul de quantité de caractéristique (10) configurée pour calculer une quantité de caractéristique relative à la région de vessie urinaire extraite pour chacune des images ultrasonores du deuxième groupe de trames par l'unité d'extraction de vessie urinaire ;
une unité d'extraction de trame candidate (11) configurée pour extraire, sur la base de la quantité de caractéristique calculée par l'unité de calcul de quantité de caractéristique, une image ultrasonore d'au moins une trame candidate qui sert de candidate soumise à la mesure parmi les images ultrasonores du deuxième groupe de trames ;
un moniteur (6) configuré pour afficher l'image ultrasonore de l'au moins une trame candidate extraite par l'unité d'extraction de trame candidate ;
un dispositif d'entrée (15) avec lequel un utilisateur effectue une opération d'entrée ;
une unité de sélection de trame de mesure (12) configurée pour sélectionner,
conformément à l'opération d'entrée effectuée par l'utilisateur via le dispositif d'entrée, une image ultrasonore d'une trame de mesure qui sert de cible soumise à la mesure, à partir de l'image ultrasonore de l'au moins une trame candidate affichée sur le moniteur ; et
une unité de mesure de volume d'urine (13) configurée pour analyser l'image ultrasonore de la trame de mesure sélectionnée par l'unité de sélection de trame de mesure pour mesurer un volume d'urine,
dans lequel l'unité de réduction du nombre de trames est configurée pour extraire une image ultrasonore à des intervalles prédéterminés dans une série temporelle parmi les images ultrasonores du premier groupe de trames et pour supprimer des images ultrasonores des trames restantes parmi les images ultrasonores du premier groupe de trames pour former le deuxième groupe de trames d'images ultrasonores, et
dans lequel l'unité de réduction du nombre de trames inclut
une unité d'estimation de similarité (31A, 31B, 31C) configurée pour estimer si des images ultrasonores de deux trames sont ou non similaires l'une à l'autre, et
une unité de suppression de trames (38) configurée pour supprimer, parmi les images ultrasonores du deuxième groupe de trames, une image ultrasonore d'une trame qui est consécutive dans une série temporelle à l'image ultrasonore extraite d'une trame et qui est estimée comme étant similaire à l'image ultrasonore extraite de ladite une trame par l'unité d'estimation de similarité.

2. Appareil de diagnostic par ultrasons selon la revendication 1, dans lequel les intervalles prédéterminés sont des intervalles d'un nombre prédéterminé de trames ou des intervalles de temps prédéterminés.

3. Appareil de diagnostic par ultrasons selon la revendication 1, dans lequel
l'unité d'estimation de similarité
inclut une unité de calcul de niveau de similarité (33) configurée pour analyser les images ultrasonores des deux trames pour calculer un niveau de similarité entre les images ultrasonores des deux trames, et
est configurée pour estimer que les images ultrasonores des deux trames sont similaires l'une à l'autre dans un cas où le niveau de similarité calculé par l'unité de calcul de niveau de similarité est supérieur ou égal à un seuil de niveau de similarité prédéterminé, et pour estimer que les images ultrasonores des deux trames sont dissemblables l'une de l'autre dans un cas où le niveau de similarité calculé par l'unité de calcul de niveau de similarité est inférieur au seuil de niveau de similarité prédéterminé.

4. Appareil de diagnostic par ultrasons selon la revendication 1, dans lequel
l'unité d'estimation de similarité
inclut une unité de calcul de rapport de surface (34) configurée pour analyser les images ultrasonores des deux trames pour calculer un rapport de surface entre des régions de vessie urinaire dans les images ultrasonores respectives des deux trames, et est configurée pour estimer que les images ultrasonores des deux trames sont similaires l'une à l'autre dans un cas où le rapport de surface calculé par l'unité de calcul de rapport de surface se situe dans une plage de rapport de surface prédéterminée, et pour estimer que les images ultrasonores des deux trames sont dissemblables l'une de l'autre dans un cas où le rapport de surface calculé par l'unité de calcul de rapport de surface se situe en dehors de la plage de rapport de surface prédéterminée.

5. Appareil de diagnostic par ultrasons selon la revendication 1, comprenant en outre :
une sonde ultrasonore (21) configurée pour transmettre un faisceau ultrasonore à un sujet et recevoir un faisceau ultrasonore provenant du sujet ; et
un capteur d'angle (35) fixé à la sonde ultrasonore et configuré pour détecter un angle de la sonde ultrasonore, dans lequel
l'unité d'estimation de similarité
inclut une unité de calcul de variation d'angle (36) configurée pour calculer, sur la base de l'angle de la sonde ultrasonore détecté par le capteur d'angle, une variation de l'angle de la sonde ultrasonore pendant l'acquisition des images ultrasonores des deux trames, et
est configurée pour estimer que les images ultrasonores des deux trames sont similaires l'une à l'autre dans un cas où la variation de l'angle calculé par l'unité de calcul de variation d'angle est inférieur à un seuil de variation d'angle prédéterminé, et pour estimer que les images ultrasonores des deux trames sont dissemblables l'une de l'autre dans un cas où la variation de l'angle calculé par l'unité de calcul de variation d'angle est supérieur ou égal au seuil de variation d'angle prédéterminé.

6. Appareil de diagnostic par ultrasons selon l'une quelconque des revendications 3 à 5, dans lequel l'unité de réduction du nombre de trames met à jour les intervalles prédéterminés, sur la base du niveau de similarité calculé par l'unité de calcul de niveau de similarité, du rapport de surface calculé par l'unité de calcul de rapport de surface ou de la variation de l'angle calculé par l'unité de calcul de variation d'angle.

7. Appareil de diagnostic par ultrasons selon l'une quelconque des revendications 1 à 6, dans lequel l'unité de réduction du nombre de trames met à jour les intervalles prédéterminés, sur la base d'une vitesse de traitement de l'appareil de diagnostic par ultrasons.

8. Appareil de diagnostic par ultrasons selon l'une quelconque des revendications 1 à 7, dans lequel l'unité de réduction du nombre de trames met à jour les intervalles prédéterminés, sur la base d'un temps nécessaire pour un balayage d'une vessie urinaire.

9. Appareil de diagnostic par ultrasons selon la revendication 1, dans lequel
l'unité de réduction du nombre de trames
inclut une unité d'estimation de similarité configurée pour estimer si des images ultrasonores de deux trames sont ou non similaires l'une à l'autre, et
est configuré pour former le deuxième groupe de trames d'images ultrasonores en utilisant des images ultrasonores de trames qui restent après suppression d'une image ultrasonore d'une trame qui est consécutive à une image ultrasonore de chaque trame parmi les images ultrasonores du premier groupe de trames et qui est estimée comme étant similaire à l'image ultrasonore de la trame par l'unité d'estimation de similarité.

10. Appareil de diagnostic par ultrasons selon la revendication 9, dans lequel
l'unité d'estimation de similarité
inclut une unité de calcul de niveau de similarité (33) configurée pour analyser les images ultrasonores des deux trames pour calculer un niveau de similarité entre les images ultrasonores des deux trames, et
est configurée pour estimer que les images ultrasonores des deux trames sont similaires l'une à l'autre dans un cas où le niveau de similarité calculé par l'unité de calcul de niveau de similarité est supérieur ou égal à un seuil de niveau de similarité prédéterminé, et pour estimer que les images ultrasonores des deux trames sont dissemblables l'une de l'autre dans un cas où le niveau de similarité calculé par l'unité de calcul de niveau de similarité est inférieur au seuil de niveau de similarité prédéterminé ; ou
dans lequel
l'unité d'estimation de similarité
inclut une unité de calcul de rapport de surface (34) configurée pour analyser les images ultrasonores des deux trames pour calculer un rapport de surface entre des régions de vessie urinaire dans les images ultrasonores respectives des deux trames, et est configurée pour estimer que les images ultrasonores des deux trames sont similaires l'une à l'autre dans un cas où le rapport de surface calculé par l'unité de calcul de rapport de surface se situe dans une plage de rapport de surface prédéterminée, et pour estimer que les images ultrasonores des deux trames sont dissemblables l'une de l'autre dans un cas où le rapport de surface calculé par l'unité de calcul de rapport de surface se situe en dehors de la plage de rapport de surface prédéterminée.

11. Appareil de diagnostic par ultrasons selon la revendication 9, comprenant en outre :
une sonde ultrasonore (21) configurée pour transmettre un faisceau ultrasonore à un sujet et recevoir un faisceau ultrasonore provenant du sujet ; et
un capteur d'angle (35) fixé à la sonde ultrasonore et configuré pour détecter un angle de la sonde ultrasonore, dans lequel
l'unité d'estimation de similarité
inclut une unité de calcul de variation d'angle configurée pour calculer, sur la base de l'angle de la sonde ultrasonore détecté par le capteur d'angle, une variation de l'angle de la sonde ultrasonore pendant l'acquisition des images ultrasonores des deux trames,
et
est configurée pour estimer que les images ultrasonores des deux trames sont similaires l'une à l'autre dans un cas où la variation de l'angle calculé par l'unité de calcul de variation d'angle est inférieur à un seuil de variation d'angle prédéterminé, et pour estimer que les images ultrasonores des deux trames sont dissemblables l'une de l'autre dans un cas où la variation de l'angle calculé par l'unité de calcul de variation d'angle est supérieur ou égal au seuil de variation d'angle prédéterminé.

12. Procédé de contrôle d'un appareil de diagnostic par ultrasons, comprenant :
réduire le nombre des images ultrasonores de certaines trames parmi les images ultrasonores d'un premier groupe de trames pour former un deuxième groupe de trames d'images ultrasonores, le deuxième groupe de trames étant constitué d'un nombre réduit de trames par rapport au premier groupe de trames d'images ultrasonores ;
extraire une région de vessie urinaire à partir de chacune des images ultrasonores du deuxième groupe de trames ;
calculer une quantité de caractéristique relative à la région de vessie urinaire extraite pour chacune des images ultrasonores du deuxième groupe de trames ;
extraire, sur la base de la quantité de caractéristique, une image ultrasonore d'au moins une trame candidate qui sert de candidate soumise à la mesure parmi les images ultrasonores du deuxième groupe de trames ;
afficher l'image ultrasonore de l'au moins une trame candidate sur un moniteur ;
sélectionner, conformément à une opération d'entrée effectuée par un utilisateur, une image ultrasonore d'une trame de mesure qui sert de cible soumise à la mesure, à partir de l'image ultrasonore de l'au moins une trame candidate affichée sur le moniteur ;
analyser l'image ultrasonore sélectionnée de la trame de mesure pour mesurer un volume d'urine ; et
extraire, par une unité de réduction du nombre de trames (8), une image ultrasonore à des intervalles prédéterminés dans une série temporelle parmi les images ultrasonores du premier groupe de trames et supprimer des images ultrasonores des trames restantes parmi les images ultrasonores du premier groupe de trames pour former le deuxième groupe de trames d'images ultrasonores,
dans lequel l'unité de réduction du nombre de trames inclut une unité d'estimation de similarité (31A, 31B, 31C) et une unité de suppression de trames (38), et le procédé comprenant en outre :
estimer, par l'unité d'estimation de similarité (31A, 31B, 31C), si des images ultrasonores de deux trames sont ou non similaires l'une à l'autre, et
supprimer, par l'unité de suppression de trames (38), parmi les images ultrasonores du deuxième groupe de trames, une image ultrasonore d'une trame qui est consécutive dans une série temporelle à l'image ultrasonore extraite d'une trame et qui est estimée comme étant similaire à l'image ultrasonore extraite de ladite une trame par l'unité d'estimation de similarité.

13. Processeur pour un appareil de diagnostic par ultrasons, le processeur étant configuré à :
réduire le nombre des images ultrasonores de certaines trames parmi les images ultrasonores d'un premier groupe de trames pour former un deuxième groupe de trames d'images ultrasonores, le deuxième groupe de trames étant constitué d'un nombre réduit de trames par rapport au premier groupe de trames d'images ultrasonores ;
extraire une région de vessie urinaire à partir de chacune des images ultrasonores du deuxième groupe de trames ;
calculer une quantité de caractéristique relative à la région de vessie urinaire extraite pour chacune des images ultrasonores du deuxième groupe de trames ;
extraire, sur la base de la quantité de caractéristique, une image ultrasonore d'au moins une trame candidate qui sert de candidate soumise à la mesure parmi les images ultrasonores du deuxième groupe de trames ;
afficher l'image ultrasonore de l'au moins une trame candidate sur un moniteur ;
sélectionner, conformément à une opération d'entrée effectuée par un utilisateur, une image ultrasonore d'une trame de mesure qui sert de cible soumise à la mesure, à partir de l'image ultrasonore de l'au moins une trame candidate affichée sur le moniteur ;
analyser l'image ultrasonore de la trame de mesure sélectionnée par l'utilisateur pour mesurer un volume d'urine ; et
extraire, par une unité de réduction du nombre de trames (8), une image ultrasonore à des intervalles prédéterminés dans une série temporelle parmi les images ultrasonores du premier groupe de trames et supprimer des images ultrasonores des trames restantes parmi les images ultrasonores du premier groupe de trames pour former le deuxième groupe de trames d'images ultrasonores,
dans lequel l'unité de réduction du nombre de trames inclut une unité d'estimation de similarité (31A, 31B, 31C) et une unité de suppression de trames (38), et le processeur est en outre configuré pour :
estimer, par l'unité d'estimation de similarité (31A, 31B, 31C), si des images ultrasonores de deux trames sont ou non similaires l'une à l'autre, et
supprimer, par l'unité de suppression de trames (38), parmi les images ultrasonores du deuxième groupe de trames, une image ultrasonore d'une trame qui est consécutive dans une série temporelle à l'image ultrasonore extraite d'une trame et qui est estimée comme étant similaire à l'image ultrasonore extraite de ladite une trame par l'unité d'estimation de similarité.
